Europäisches Patentamt

European Patent Office

Office européen des brevets

(11) Publication number : **0 494 502 A1**

# (12) EUROPEAN PATENT APPLICATION

(21) Application number : **91311129.0**

(22) Date of filing : **29.11.91**

(51) Int. Cl.$^5$ : **G01N 33/68, C07K 3/00**

(30) Priority : **30.11.90 JP 329895/90**
**15.07.91 JP 173690/91**

(43) Date of publication of application :
**15.07.92 Bulletin 92/29**

(84) Designated Contracting States :
**DE FR GB SE**

(71) Applicant : **SAGAMI CHEMICAL RESEARCH CENTER**
**11-1, Marunouchi 1-chome Chiyoda-ku**
**Tokyo (JP)**
(71) Applicant : **Tosoh Corporation**
**4560, Kaisei-cho**
**Shinnanyo-shi, Yamaguchi-ken (JP)**
(71) Applicant : **Nippon Mining Co., Ltd.**
**10-1, Toranomon 2-chome, Minato-ku**
**Tokyo 105 (JP)**

(72) Inventor : **Numao, Naganori**
**9-2 Minamidai 1-chome**
**Sagamihara-shi, Kanagawa (JP)**
Inventor : **Kidokoro, Shunichi**
**9-2 Minamidai 1-chome**
**Sagamihara-shi, Kanagawa (JP)**

(74) Representative : **Stuart, Ian Alexander et al**
**MEWBURN ELLIS 2 Cursitor Street**
**London EC4A 1BQ (GB)**

(54) **Method for surmising functional site in physiologically active polypeptide or polynucleotide.**

(57)    A method is provided wherein based only on the amino acid sequence of an objective polypeptide or protein or the nucleotide sequence of an objective polynucleotide, its functional site or region such as catallytically active site or binding site can be surmised and generally be applied to a wide range of polypeptides or proteins or polynucleotides.

(1) Plurality of polypeptides or proteins whose amino acid sequence and a functional site are already known (hereinafter referred to as "reference polypeptides") are selected, (2) the relations between the functional sites and amino acid sequences, common to these polypeptides are extracted, and a law is induced from the relations, and (3) the law is applied to a polypeptide the amino acid sequence of which is known but the functional site or region of which is not known (test polypeptide).

EP 0 494 502 A1

## BACKGROUND OF THE INVENTION

### Field of the Invention

The present invention relates to a method for surmising a functional site or region of a physiologically active polypeptide, protein or a polynucleotide. If it is possible to surmise, with a high probability, a functional site or region of the physiologically active polypeptide, protein or polynucleotide, it is extremely industrially useful because such an information can give a deep insight when we want to modify a proper position or region of the polypeptide or proteins to construct more useful one with desired functions. This invention is particularly useful for creating novel and useful polypeptides or proteins or polynucleotides by such a method of M. Ballivet et al. (Literature 10) to modify their functional regions.

### Related Art

Although various methods have been proposed to surmise the functional site or region of a physiologically active polypeptide or protein (Literatures 1 to 8 and 11 to 12), a method has not been known which can surmise the functional site or region of an objective polypeptide or protein using the information on the amino acid sequence thereof alone and which can generally be applied to every polypeptide or protein existing in nature. Namely, it is extremely difficult to surmise the catalytically active site of enzymes or the binding region of receptors or lymphokines or the like except such a method based on homologous sequences (homology) (Literatures 5 and 12). Further, since in proteins there are generally many functional sites or regions involved in the molecular recognition and response in regions other than homologous sequences (homology), it is difficult to find them solely by the homologous sequences (homology).

## SUMMARY OF THE INVENTION

The present invention aims at providing a method that can surmise the functional site or region of an objective polypeptide or protein or polynucleotide based on the amino acid sequence or nucleotide sequence alone, and moreover can generally be applied to a wide range of polypeptides or proteins or polynucleotides.

The above problem is solved by the method of this invention which comprises (1) selecting plural polypeptides or proteins (hereinafter referred to as "reference polypeptides") whose amino acid sequence and active sites have been already confirmed, (2) extracting the relations between the active sites and the amino acid sequences, common to these reference polypeptides, and deriving a law from the relations, and (3) applying the law to a polypeptide (hereinafter reffered to as "test polypeptide") whose amino acid sequence is known but whose the functional site or region is not known.

This method can also be used for surmising the functional site or region of a polynucleotide such as the ribozyme which we called.

## BRIEF DESCRIPTION OF DRAWINGS

Fig. 1 denotes a surmised pattern of the functional region of bovine pancreas ribonuclease.

Fig. 2 denotes a surmised pattern of the functional region of canine pancreas cationic trypsinogen.

Fig. 3 denotes a surmised pattern of the functional region of rat preprocarboxypeptidase A.

Fig. 4 denotes a surmised pattern of the functional region of human carbonic anhydrase I.

Fig. 5 denotes a surmised pattern of the functional region of human Cu/Zn superoxide dismutase.

Fig. 6 denotes a surmised pattern of the functional region of chicken triosephosphate isomerase.

Fig. 7 denotes a surmised pattern of the functional region of human type I alcohol dehydrogenase.

Fig. 8 denotes a surmised pattern of the functional region of Escherichia coli glutathione reductase.

Fig. 9 denotes a surmised pattern of the functional region of rat liver catalase.

Fig. 10 denotes a surmised pattern of the functional region of bovine lysozyme C.

Fig. 11 denotes a surmised pattern of the functional region of Kiwi fruit actinidine.

Fig. 12 denotes a surmised pattern of the functional region of human glyceraldehyde-3-phosphate dehydrogenase.

Fig. 13 denotes a surmised pattern of the functional region of bovine pancreas phospholipase A2.

Fig. 14 denotes a surmised pattern of the functional region of B. stearothermophilus thermolysin.

Fig. 15 denotes a surmised pattern of the catalytically functional region of tobacco ring spot virus (TRSV).

Where sum means the probabilities of the functional site such as the catalytically active site or binding site of proteins.

DETAILED DESCRIPTION

Physiologically active polypeptides or proteins in this invention include all peptide substances having some physiological activity, and comprehensive substances such as enzymes, hormones and various interleukins are included. An functional site means a site consisting of one or a small number of amino acids required for these physiologically active polypeptides to express their functions, and for example, in enzymes their catallytical active site is meant and in other polypeptides the binding site is meant. Further, an functional region means a region which is involved in the expression of the physiological activities of a physiologically active polypeptide and has some extent.

This invention is specifically described below.

The "reference polypeptide" in the invention can be any polypeptide whose amino acid sequence and the functional sites are known. The number thereof is not particularly critical and it is thought that the higher the number, the more accurately a method for surmising the functional site can be, and about 10 reference polypeptides more are sufficient. In the specific examples of the invention, 17 enzymes shown in Table 1 (Literature 7) were used.

Table 1

| No. | Enzyme name | Length of the amino acid sequence | Active site | ID name of PDB |
|---|---|---|---|---|
| 1 | Ribonuclease | 124 | 12, 41[*], 119 | 1RN3 |
| 2 | Trypsinogen | 223 | 40, 84, 117 | 2PTN |
| 3 | Carboxypeptidase A | 307 | 127, 248[*], 270 | 5CPA |
| 4 | Carbonic anhydrase I | 260 | 106, 199 | 2CAB |
| 5 | L-lactate dehydrogenase | 329 | 99, 169, 193 | 4LDH |
| 6 | Superoxide dismutase | 151 | 61, 141 | 2SOD |
| 7 | Penicillopepsin | 323 | 33, 213 | 2APP |
| 8 | Triosephosphate isomerase | 247 | 164 | 1TIM |
| 9 | Alcohol dehydrogenase | 374 | 48, 51 | 4ADH |
| 10 | Glutathione reductase | 478 | 58, 63, 467 | 2GRS |
| 11 | Rhodanese | 293 | 186, 247, 249 | 1RHD |
| 12 | Catalase | 506 | 74, 147 | 8CAT |
| 13 | Lysozyme C | 130 | 35, 53 | 1LZI |
| 14 | Actinidine | 220 | 25, 162 | 2ACT |
| 15 | Glyceraldehyde 3-phosphate dehydrogenase | 333 | 148, 175 | 1GPD |
| 16 | Prophospholipase A2 | 130 | 55, 106[*] | 2BPZ |
| 17 | Thermolysin | 316 | 143, 231 | 3TLN |

The position * was quoted from BION/swiss-prot database.

Then, in the amino acid sequence of reference polypeptides selected in the above way, a region (supposed functional region) surrounded by plural amino acids upstream and downstream from the position of a catallytical active site are extracted. The number of amino acids in the supposed function region are not particularly limited, and for example, about 15 is sufficient.

Then, amino acid sequence patterns represented by the following formula (I):

$$X_1(Z)_nX_2 \qquad (I)$$

are supposed using 20 amino acids existing in natural proteins. This amino acid sequence pattern is referred to as "supposed amino acid sequence pattern" in the invention. In the formula (I), $X_1$ and $X_2$ are the same or different, and specific amino acids selected from the 20 amino acids.

In this case, although it is desirable to use as amino acids all of the 20 amino acids usually existing in proteins, namely glycine (Gly; G), alanine (Ala; A), valine (Val; V), leucine (Leu; L), isoleucine (Ile; I), Serine (Ser; S), threonine (Thr; T), aspartic acid (Asp; D), glutanic acid (Glu; E), asparagine (Asn; N), glutamine (Glu; Q), lysine (Lys; K), arginine (Arg; R), cysteine (Lys; C), methionine (Met; M), phenylalanine (Phe: F), tyrosine (Tyr; Y), tryptophan (Trp; W), histidine (His; H) and proline (Pro; P), it is possible to omit a small number of amino acids whose occuring frequencies in natural proteins is very low and which do not have a significant influence on the method of the invention.

$Z_s$ are the same or different, and mean any unspecified amino acids in the above amino acids. n is 0 or an integer of 1 to above 30. Although the upper limit of n is not particularly critical, 30 is sufficient. The amino acid $X_1$ is referred to as "reference amino acid" for the sake of descriptive convenience.

Then, plural or all amino acid sequence patterns satisfying the requisite of the formula (I) are supposed. For example, when it is supposed that $X_1$ and $X_2$ are selected from 20 amino acids and the number of n, i.e., the number of an unspecific amino acid Z, moves from 0 to 30, the amino acid sequence patterns of 20 x 20 x 31 = 12,400 can be obtained.

In the above, however, the upper limit of n is not necessarily 30, and for example, when the upper limit of n is 2, namely n is 0, 1 or 2, the number of the supposed amino acid sequence patterns becomes 20 x 20 x 3 = 1,200. Thus, sequence patterns such as, for example, Gly Gly, Gly Ala, Gly Val, ... (n = 0); Gly Z Gly, Lys Z Ala, Gly Z Val, ... (n = 1); and Gly Z Z Gly, Gly Z Z Ala, Gly Z Z Val, ... (n = 2) are constructed. In the above Z may be any of the unspecified 20 amino acids.

Then, the reference amino acid sequences is scanned by each of these specified amino acid sequence pattern while the latter is moved along with the former. The sum of the frequency such that the $X_1$ and $X_2$ of the supposed amino acid sequence pattern agree with the amino acids at the positions corresponding thereto in the reference amino acid sequences is determined. The supposed amino acid sequence patterns in both the "supposed functional regions" and the region other than the functional regions (this is referred to as "supposed non-functional region") in the reference amino acid sequences are divided.

This operation is carried out on, for example, 10 or more of reference amino acid sequences. Although the total number of the supposed amino acid sequence patterns used in this operation is not particularly restriced, 20 x 20 x 13 or more (i.e., $n \geq 12$) is desirable.

The above operation, to our surprise, gave present inventors a fact that the used supposed amino acid sequence patterns could be classified into ones specifically according with or favoring for the supposed functional regions of the reference amino acid sequences, and the others specifically according with or favoring for the supposed non-functional regions thereof (incidentally, there were also a small number of supposed amino acid sequence patterns not specific for both regions).

When, as a typical example, supposed amino acid sequence patterns with the occurring frequencies more than 20 times were selected from the amino acid sequences of the 17 reference polypeptides under the conditions of 20 x 20 x 13 supposed amino acid sequence patterns (n = 0 to 12), supposed amino acid sequence patterns whose reference amino acid $X_1$ is Cys, His, Arg, Ala, Gly, Ser, Thr or Asn were favored for the supposed functional regions, and on the other hand, specified amino acid sequence patterns whose reference amino acid $X_1$ is Asp, Glu, Phe, Lys, Leu, Pro or Tyr were favored for the supposed non-functional regions.

Some of supposed amino acid sequence patterns corresponding to reference amino acids $X_1$ could not be classified into either a region by the above classification criterion, because of a low occurring frequency of the same amino acid.

Then, with respect to the supposed amino acid sequence patterns classified into any of the supposed functional regions and the supposed non-functional regions, the supposed amino acid sequence patterns having the same specified reference amino acid $X_1$ are individually collected in each region, and then superposed the reference amino acid to align one amino acid sequence pattern. These are particularly related to either the supposed functional regions or the supposed non-functional regions. Therefore, these are referred to as "related amino acid sequence pattern".

By this operation, the following "related amino acid sequence patterns" were obtained in the above specific examples of the invention. Namely, as functional region-related amino acid sequence patterns:

1. <u>Cys</u> (Gly/Ser) Ala Z Gly Z (Gly/Ala) (SEQ ID:1)

2. <u>His</u> Z Z Ser (Gly/Ser) Z Gly Z Gly Z Gly
   (SEQ ID:2)

3. <u>Arg</u> Ser Z Z Z Ala Z Ser Z Z (Gly/Ala/Ser)
   (SEQ ID:3)

4. <u>Ala</u> Z Ser Z Z Z Z Gly Z Z Gly Z Ala Z
   (SEQ ID:4)

5. <u>Gly</u> (Gly/Ser) Z Z Z Ser Gly Ala Ser Gly
   (Ala/Gly) Gly (Gly/Ser) (SEQ ID:5)

6. <u>Ser</u> (Gly/Ser) Ala Ser Z Gly Ser Z Gly Z Ser Z
   Gly (SEQ ID:6)

7. <u>Thr</u> (Gly/Ala) Ala Z Ala (Ala/Ser) Z Ala
   (Ala/Ser) (Gly/Ser) (SEQ ID:7)

8. <u>Asn</u> Z Z Ser (Gly/Ala) Ala Z (Ser/Ala) Gly
   (Ser/Ala) Z Z Ser (SEQ ID:8)

As non-functional region-related amino acid sequence patterns

9. <u>Asp</u> Pro (Leu/Lys) Z Leu Z Z Val Lys Leu Leu
   Leu (Gly/Lys) (SEQ ID:9)

10. <u>Glu</u> Z Z Z Z Lys Z Leu Leu (Asp/Leu) (Gly/Asp)
    Z (Gly/Lys) (SEQ ID:10)

11. <u>Phe</u> (Gly/Leu) Asp Asp (Gly/Val) Z (Val/Asp)
    Val Z Z (Gly/Leu) Val Gly Asp (SEQ ID:11)

12. <u>Lys</u> Leu (Asp/Lys) Z Z Gly Leu Gly (Asp/Lys)
    (Val/Leu) Gly Leu Asp Leu (SEQ ID:12)

13. <u>Leu</u> Lys Asp Z (Gly/Asp) Z Z Z Leu (Asp/Lys)
    (Asp/Val) (Asp/Lys) (Val/Leu) (Lys/Val)
    (SEQ ID:13)

14. <u>Pro</u> Asp Lys Gly Z Z Z Z (Val/Gly) (Gly/Lys)
    Lys Val (Gly/Lys) Leu (SEQ ID:14)

15. <u>Tyr</u> Val Z Leu (Val/Leu) Val Z Z Leu Z Asp
    (SEQ ID:15)

In the above related amino acid sequence patterns, the underlined amino acids are reference amino acids $X_1$.

Either amino acid deviced by the slash in the parentheses represents one of $X_2$. For example His can pair with Gly or Ser in (SEQ ID:2), namely His ZZZ Gly or His ZZZ Ser.

Then, it is necessary to prepare a "discrimination criterion" in order to surmise, utilizing the "related amino acid sequence patterns", the active sites of a polypeptide (test polypeptide) whose amino acid sequence is known but whose active sites are not known.

This discrimination criterion is induced by applying, to a reference amino acid sequence whose active site is already known, the above "related amino acid sequence patterns", namely the functional region-related amino acid sequence patterns or non-functional region-related amino acid sequence patterns, or both of them.

EP 0 494 502 A1

As a result, a condition by which the active site in the reference amino acid sequence is most certainly related to the related amino acid sequence can be induced.

For example, based on the related amino acid sequence patterns specific for the supposed functional region, is prepared a symmetrically related amino acid sequence pattern situated the reference amino acid at the center (this is referred to as a "symmetrically related amino acid sequence pattern"). For example, when the related amino acid sequence pattern (1) is

```
Cys (Gly/Ser) Ala Z Gly Z (Gly/Ala),
(SEQ ID:16),
```

its symmetrical related amino acid sequence pattern is

```
(Gly/Ala) Z Gly Z Ala (Gly/Ser) Cys (Gly/Ser)
Ala Z Gly Z (Gly/Ala) (SEQ ID:17).
```

Such a "symmetrically related amino acid sequence pattern" can be prepared from each related amino acid sequence pattern.

Then, this "symmetrically related amino acid sequence pattern" moved from N-terminal to C-terminal of the reference amino acid sequence to count the sum of number of amino acid overlapped completely. For example, in the range respectively of 5 to 10 amino acids upstream and downstream of the reference amino acid, the number of "the same amino acids", which are amino acids other than the reference amino acid, can be counted by the comparison of the "symmetrically related amino acid sequence pattern" with the reference amino acid sequence.

This operation is carried out on each symmetrically related amino acid sequence pattern. For specification of the active site in the reference sequence, the requisite of "the number of the same amino acids" and "the number of symmetrically related amino acid sequence patterns" are determined.

For example, in a typical example of the invention, the above requisites were determined as follows.

The first requisite:

A site or region is that, in the range of 10 amino acids in both upstream and downstream of the reference amino acid, "the number of the same amino acids" on "symmetrically related amino acid sequence patterns" whose reference amino acids are Cys, His, Arg, Ala, Gly, Ser, Thr and Asn are more than 2, 2, 2, 2, 4, 3, 3 and 3 respectively, and that the number of "symmetrically related amino acid sequence patterns" satisfying the requisite is 3 or more;

The second requisite:

A site or region is that in the range of 10 amino acids in both upstream and downstream of the reference amino acid, "the number of the same amino acids" on "symmetrically related amino acid sequence patterns" whose reference amino acids are Cys, His, Arg, Ala, Gly, Ser, Thr and Asn are 2, 2, 2, 2, 4, 3, 3 and 3 respectively, and that the number of "symmetrically related amino acid sequence patterns" satisfying the requisite is 2 or more, and that in the ranges of 5 amino acids in both upstream and downstream of the reference amino acid, "the number of the same amino acids" of "symmetrically related amino acid sequence patterns" containing Cys as the reference amino acid is 3 or more, or two more Cysteines exist in the above range.

The third requisite:

A site or region is that in the range of 10 amino acids in both upstream and downstream of the reference amino acid, "the number of the same amino acids" on "symmetrically related amino acid sequence patterns" whose reference amino acids are Cys, His, Arg, Ala, Gly, Ser, Thr and Asn are 2, 2, 2, 2, 4, 3, 3 and 3 respectively, and that the number of "symmetrically related amino acid sequence patterns" is 2 or more, and that in the range of 5 amino acids in both upstream and downstream of the reference amino acid, none of "the number of the same amino acids" on "symmetrically related amino acid sequence patterns" whose reference amino acids are Asp, Glu, Phe, Lys, Leu, Pro and Tyr are composed of 2, 2, 3, 4, 3, 3 and 2 respectively.

7

<u>The fourth requisite:</u>

A site or region is that in the range of 10 amino acids in both upstream and downstream of the reference amino acid, "the number of same amino acids" of the "symmetrically related amino acid sequence patterns" whose reference amino acids are Cys, His, Arg, Ala, Gly, Ser, Thr and Asn, is not 0, and that such patterns emerge in a number of 3 or more in a row;

<u>The fifth requisite:</u>

A site or region is that in the range of 10 amino acids in both upstream and downstream of the reference amino acid, "the number of the same amino acids" of the "symmetrically related amino acid sequence patterns" whose reference amino acids are Cys, His, Arg, Ala, Gly, Ser, Thr and Asn respectively, is not 0, and "the number of the same amino acids" of the "symmetrically related amino acid sequence patterns" whose reference amino acids are Asp, Glu, Phe, Lys, Leu, Pro and Tyr respectively, is not 0, and that such patterns emerge in a number of 5 or more in a row;

<u>The sixth requisite:</u>

A site or region is that in the range of 10 amino acids in both upstream and downstream of the reference amino acid, "the number of the same amino acids" of the "symmetrical related amino acid sequence patterns" whose reference amino acids are Asp, Glu, Phe, Lys, Leu, Pro and Tyr, is 0, and that such patterns concentrate or these amino acids.

In order to surmise an active site in the test polypeptide, first, the "symmetrically related amino acid sequence patterns" is superposed on the amino acid sequence of the test polypeptide, and that in the range of 5 to 10 amino acids in both upstream and downstream of the reference amino acid, "the number of the same amino acids", which are amino acids other than the reference amino acid, are counted.

By applying the above first requisite to this result, a functional site or region in the amino acid sequence of the test polypeptide is surmised. Consequently, when a region of a reasonably narrow range can not be specified, the second condition can be then applied. If necessary, the third condition, the fourth condition, etc. can be applied in this order, and the above operation can be repeated until an action region of a reasonably narrow range can be surmised.

Incidentally, the above "related amino acid sequence patterns" are classified into ones related to the functional region and the others related to the non-functional region, and the amino acid $X_1$ and the amino acid $X_2$ are extracted on both regions to obtain the following results.

Supposed functional region-related patterns

$X_1$ ... Ala, Cys, Gly, His, Asn, Arg, Ser, Thr

$X_2$ ... Gly, Ala, Ser

Supposed non-functional region-related patterns

$X_1$ ... Asp, Glu, Phe, Lys, Leu, Pro, Tyr

$X_2$ ... Leu, Lys, Gly, Asp, Val

The inventors investigated the relation between the above $X_1$ and $X_2$ from a view of new point. For example, when Ala (alanine) of $X_2$ of the functional region-related patterns is directed to, one of the codons (Although a codon usually means one having as a unit a sequence consisting of three of four kinds of bases composing a mRNA, this is conveniently applied to DNA in this specification. This is also the case with "sense" and "antisense".) is "GCT".

The antisense against this codon is "CGA", and when this antisense is read in the direction of $5' \rightarrow 3'$, "AGC" is obtained whereby Ser (serine) is coded. Further, when "GCT", a codon of Ala is read in the reverse direction $(3' \rightarrow 5')$, "TCG" is obtained whereby Ser (serine) is encoded, and when the antisense "CGA" is read in the reverse direction $(3' \rightarrow 5')$, a codon encoding Arg (arginine) is obtained. All these amino acids (Ser and Arg) exist in the amino acids of $X_1$ of the functional region-related patterns.

Similar relations exist on Ser of $X_2$ of the functional region-related patterns, too. Therefore, it is surmised that with respect to the amino acids of $X_1$ and the amino acids of $X_2$, codons encoding them closely correspond to antisense. Consequently, by utilizing this relation, it is possible to efficiently surmise an active site in the test polypeptide.

Therefore, the second method for surmising an functional site or region in the amino acid sequence of a test polypeptide uses the above finding.

For this purpose, at first, an amino acid sequence pattern represented by the following formula (II):

$$X_1' \, (Z)_m \, X_2' \qquad (II)$$

is supposed. This amino acid sequence pattern is referred to as a secondary amino acid sequence pattern for the sake of convenience. In the above formula (II), $X_1'$ and $X_2'$ are specific amino acids selected from 20 amino acids naturally existing in proteins, and $X_1'$ and $X_2'$ are related to the above through their sense and antisense.

In this sense, the above restriction requisite is essentially, different from supposed amino acid sequences denoted by $X_1 (Z)_n X_2$ [i.e., formula (I)]. $Z_s$ are the same or different unspecific amino acids, and m is 0 or an integer of 1 to about 30. However, since $X_1'$ and $X_2'$ are related as above, it is sufficient to use m of 0 or 1, i.e., $X_1' X_2'$ and $X_1' Z X_2'$.

Then these secondary amino acid sequence patterns are applied to the reference amino acid sequences, multivariate analysis is carried out to lead to a discriminant function capable of discriminating an active site or region from other.

Then, the thus obtained discriminant function can be applied to the amino acid sequence of the test polypeptide to surmise an active site or region in the amino acid sequence.

As the first specific example of the invention, repetitive sequence patterns extracted from 160 kinds of secondary amino acid sequence patterns coming under the formulae $X_1' X_2'$ and $X_1' Z X_2'$ (Z is selected from 20 kinds of amino acids), and other 102 kinds of patterns (the related amino acid sequence patterns, doublet sequence patterns, in consideration of a substitution frequency constant, etc.) were prepared, and these were applied to the above 17 kinds of the reference amino acid sequences to derive a discriminant function for surmising an active site or region.

In this connection, when this discriminant function was applied to the above reference amino acid sequences whose active site was known, it was possible to distinguish active from non-active regions by a discrimination percentage of 84%. Preferential amino acid sequence patterns used in the discriminant function are shown below.

Namely, they are 1. Ala Z Cys 2. Gly Ser
3. Val Z His 4. Val His 5. Arg Pro 6. Thr Z Arg
7. Val Tyr 8. Leu Asn 9. Gly Thr 10. Gly Z Ser
11. Ala Arg 12. Ser Arg 13. Ser Ser 14. Leu Z Gln
15. $Z_1 Z_2 \geqq 1.8$ 16. Leu Z Asp 17. Val Gln 18. Met Z Tyr
19. Met Tyr 20. Ile Z Asp 21. Ile Asn 22. Thr Arg
23. ab $(X)_n$ ba, etc.

(wherein Z is any of the unspecified 20 kinds of amino acids. Further, $Z_1$ and $Z_2$ are amino acid sequences sequenced in tandem in the amino acid sequences, and means a combination such that the total of their substitution frequency values is 1.8 or more. The substitution frequency value was based on Literature 9. n is an integer of 0 to 30, and a and b are any of the 20 kinds of amino acids.)

In order to verify the effectiveness of the method of the invention established as above, the method of the invention was applied to many polypeptides whose amino acid sequence and an active site were known (polypeptide other than the above reference polypeptides), and the resulting results were compared with the actual active sites identified. The results are shown in Table 2.

Table 2 shows the results obtained on many enzymes, and therein, "hit rate" represent the ratio of the numbers of actual but position and the number of position surmised by the method of the invention, and "recovery rate" represents the ratio of the number of actually recoveried active sites and the number of whole active sites existing in enzymes. Table 3 shows the results of the surmised efficiency in the case of active region (i.e., binding region) of proteins other than enzymes.

<u>Table 2</u>

| | Various enzymes | Number of enzyme | Number of amino acid | Hit rate | Recovery rate |
|---|---|---|---|---|---|
| 1) | Hydrolase | | | | |
| | Serine protease | 9 | 3600 | 17/38 | 17/27 |
| | Serine esterase | 4 | 2027 | 2/14 | 2/6 |
| | Aspartyl protease | 6 | 2311 | 8/14 | 8/12 |
| | Metalloprotease | 5 | 2782 | 1/21 | 1/6 |
| | Thiolprotease | 5 | 1986 | 6/12 | 6/10 |
| | Glycosidase | 5 | 3051 | 1/22 | 1/6 |
| | Carboxypeptidase | 4 | 2073 | 4/18 | 4/7 |
| | Nuclease | 2 | 445 | 1/2 | 1/5 |
| | Others | 6 | 2524 | 4/20 | 4/6 |
| 2) | Lyase | 4 | 1749 | 5/18 | 5/5 |
| 3) | Transferase | 6 | 2463 | 5/17 | 5/8 |
| 4) | Oxidoreductase | 5 | 2247 | 5/18 | 5/8 |
| 5) | Isomerase | 3 | 1845 | 2/14 | 2/5 |
| 6) | Other | 4 | 1606 | 2/13 | 2/5 |
| 7) | Inhibiting substance | 2 | 817 | 2/4 | 2/2 |
| | | 70 | 31526 | 65/245 | 65/118 |
| | | | | 27% | 55% |

Table 3

|  | Physiologically active polypeptide | Number of amino acid | Hit rate | Recovery rate |
|---|---|---|---|---|
| 1 | PAI-1 | 402 | 1/2 | 1/1 |
| 2 | Angiotensinogen | 484 | 1/3 | 1/1 |
| 3 | CD4 | 458 | 1/5 | 1/1 |
| 4 | HIV envelope gp120 | 856 | 0/4 | 0/1 |
| 5 | Poliovinus·capsid | 881 | 1/5 | 1/1 |
| 6 | Acetylcholine receptor | 461 | 2/4 | 2/2 |
| 7 | Interleukin 2 | 153 | 1/1 | 2/5 |
| 8 | Insulin receptor | 1382 | 2/10 | 2/3 |
| 9 | Insulin | 110 | 2/2 | 2/2 |
| 10 | Calcitonin | | | |
|  | (human) | 141 | 1/1 | 1/1 |
|  | (salmon I) | 136 | 1/1 | 1/1 |
| 11 | TNF-$\alpha$ | 233 | 1/3 | 1/1 |
|  |  | 5697 | 14/41 | 15/20 |
|  |  |  | 34% | 75% |

As is apparent from Table 2 and 3, according to the method of the invention, 27% of the catalytically active sites actually existing in the enzymes were hit, and in other physiologically active peptides, 34% of the actually reported binding sites were hit.

The above method can be applied to every physiologically active polypeptide whose amino acid sequence is known, and further, when the nucleic acid sequence to encode the amino acid sequence is known, it is also possible to search the amino acid sequence supposed when its sense sequence is read in the reverse direction of $3' \to 5'$, and still further, it is also possible to surmise the active site or region by searching the amino acid sequence supposed when the antisense sequence against the code sequence is progressively read in the direction of $5' \to 3'$, or the amino acid sequence supposed when the antisense is read in the reverse direction of $3' \to 5'$.

In order to verify this point, out of 17 kinds of enzymes, the active site of 14 kinds of enzymes whose base sequence was entried in BION/genbank database (in this connection, on the enzymes from different species, their active sites were identified based on comparison with homologous sequence (Table 1)) was surmised under the conditions of investigating the amino acid sequences read in the direction of $5' \to 3'$ (N; sense, $5' \to 3'$), $3' \to 5'$ (C; antisense, $3' \to 5'$), $5' \to 3'$ (IC; antisense, $5' \to 3'$), and $3' \to 5'$ (R; sense, $3' \to 5'$). The results are shown in the following Table 4 to Table 6.

Table 4

| | | N (sense 5' → 3') | C (antisense 3' → 5') | IC (antisense 5' → 3') | R (sense 3' → 5') |
|---|---|---|---|---|---|
| 1. | Ribonuclease (bovine) (active site: 38,67,125) | 53,116 | 80 | 51 | 23,73 |
| 2. | Trypsinogen (canine) (active site: 63,107,200) | 59,119,212 | 54,123,234 | 49,118,187 | 50,122,199 |
| 3. | Carboxypeptidase A (rat) (active site: 273,358,380) | 15,179,226, 365,396 | 27,84,168,231, 381 | 25,70,172,217, 271,349,404 | 22,179,271,328, 395 |
| 4. | Carbonic anhydrase I (human) (active site: 107,200) | 119,196 | 18,21,70,117, 173 | 43,93,186,248 | 251 |
| 6. | Superoxide dismutase (human) (active site: 64,144) | 63,125 | 115 | 55,122 | 81 |

EP 0 494 502 A1

Table 5

| | | N (sense 5' → 3') | C (antisense 3' → 5') | IC (antisense 5' → 3') | R (sense 3' → 5') |
|---|---|---|---|---|---|
| 8. | Triosephosphate isomerase (chicken) (active site: 165) | 35,109,179 | 106,220 | 10,86,173 | 42,53,94,182 |
| 9. | Alcohol dehydrogenase (human) (active site: 49,52) | 51,125,207, 304 | 169,241,300 | 14,52,135,216, 290,333 | 119,202,327 |
| 10. | Glutathione reductase (Escherichia coli) (active site: 42,47,439) | 12,169,260, 371,441 | 12,130,370, 440 | 17,69,166,207, 306,412 | 18,57,165,205, 289,350,436 |
| 12. | Catalase (rat) (active site: 75,148) | 79,132,415 | 139,351,441 | 21,154,201 | 47,165,220,299 |
| 13. | Lysozyme C (bovine) (active site: 53,71) | 64,124 | 42,94 | 41,133 | 16,127 |

EP 0 494 502 A1

Table 6

|  |  | N<br>(sense<br>5' → 3') | C<br>(antisense<br>3' → 5') | IC<br>(antisense<br>5' → 3') | R<br>(sense<br>3' → 5') |
|---|---|---|---|---|---|
| 14. | Actinidine (kiwi fruit)<br>(active site: 25,162) | 25,165 | 97 | 17,143,198 | 19,146,192 |
| 15. | Glyceraldehyde 3-phosphate<br>dehydrogenase (human)<br>(active site: 142,179) | 153,291 | 144,266 | 44,115,154 | 43,103,223,296 |
| 16. | Phospholipase A2 (bovine)<br>(active site: 70,121) | 21,62,118 | 16,112 | 29 | 13,131 |
| 17. | Thermolysin (bacterium)<br>(active site: 379,467) | 132,251,378,<br>462 | 16,116,264,<br>374,463,530 | 58,110,185,<br>254,317,397,<br>457,496 | 25,112,276,<br>375,446,496 |

As mentioned above, it is possible to surmise, even when the above amino acid sequences other than the natural amino acid sequences were used, the active sites with considerable frequency. Further, it is readily thought that, by a combination of these four methods, it is possible to surmise the active sites or regions with a greater efficiency.

In order to further make the surmise method of the invention more precise, the methods described above were repeated to obtain the following "related amino acid sequence patterns".

Namely, as "supposed functional region-related amino acid sequence patterns":

```
1.   Cys (Gly/Ser) Ala Z (Gly/Arg) Z (Gly/Ala)
        (SEQ ID:18)

2.   His Z Z Ser (Gly/Ser) Z Gly Z Gly Z Gly
        (SEQ ID:19)

3.   Arg Ser Z Z Cys Ala Z Ser Z Z (Gly/Ala/Ser)
        (Ser/Val) (SEQ ID:20)

4.   Ala Thr (Ser/Thr/Val/Cys) Z (Thr/Leu/Asn)
        (Thr/Asn) Cys (Gly/Thr/Asn) (Thr/Glu)
        (Ile/Asn) (Gly/Arg) (Glu/Gln) Ala
        (SEQ ID:21)

5.   Gly (Gly/Ser/Thr) Gln Tyr (Asn/His/Cys)
        (Ser/Pro/Phe) (Gly/His/Cys) Ala
        (Ser/Ile/Asn) (Gly/Thr) (Ala/Val/Gly/Arg/
        His/Tyr) Gly (Gly/Ser) Val (SEQ ID:22)

6.   Ser (Gly/Lys/Glu/Cys/Ser/Arg) Ala (Ser/Leu/
        Asn/His) (Gln/Val/His/Phe) (Gly/Thr/Val)
        (Lys/Ser/Tyr) (Asn/Gln/Arg) (Gly/Thr)
        (Asn/Ile/Glu) (Gln/Ile/Ser/Arg) Arg
        (Asn/Gly) (Ile/Glu) (SEQ ID:23)

7.   Thr (Gly/Ala) (Ala/Ile) Thr (Ala/Val/Leu)
        (Ala/Ser/Leu) (Asp/Pro/Glu) (Ala/Thr/Leu)
        (Ala/Ser/Phe) (Gly/Ser/Val) (Asp/Leu/Val)
        (Asp/Lys/Ile) (Val/Leu) (SEQ ID:24)

8.   Asn (Thr/Leu) Leu (Val/Ser) (Gly/Ala) Ala


        (Lys/Ile) (Ser/Ala/Lys) Gly (Ser/Asp/Ala)
        Z Z (Ser/Lys) (SEQ ID:25)
```

As "non-functional region-related amino acid sequence patterns":

EP 0 494 502 A1

9. <u>Asp</u> Pro (Ala/Leu/Lys) (Ile/Phe) Leu Ala Phe
Val Lys (Leu/Glu) (Ser/Leu/Glu) (Gly/Leu)
Lys Phe (SEQ ID:26)

10. <u>Glu</u> Z Ile Z Z (Lys/Phe/Ser) Z (Pro/Leu) Leu
(Asp/Leu/Ile) (Gly/Asp) (Ser/Ile)
(Gly/Lys/Phe) (SEQ ID:27)

11. <u>Phe</u> (Gly/Leu) (Asp/Ser) (Ile/Asp) (Gly/Val)
(Ser/Glu) (Val/Asp) Val Ala Z (Gly/Leu)
(Val/Ser/Thr) (Gly/Glu) Asp (SEQ ID:28)

12. <u>Lys</u> Leu (Asp/Asn/Pro/Lys/Thr) Z Z
(Gly/Glu/Ser) (Leu/Ile) (Gly/Thr)
(Asp/Lys/Thr) (Val/Leu/Pro/Asn) (Gly/Pro)
Leu (Ser/Pro/Asp/Glu/Ile) Leu (SEQ ID:29)

13. <u>Leu</u> (Lys/Phe) (Asp/Gln) (Ile/Tyr)
(Gly/Asp/Tyr) (Arg/Asn) Glu (Glu/Leu/Tyr)
(Asp/Lys/Glu/Ile) (Asp/Lys/Glu/Ile)
(Asp/Ala/Val/Phe) (Asp/Lys) (Val/Leu)
(Pro/Lys/Val) (SEQ ID:30)

14. <u>Pro</u> (Asp/Ala/Ile) Lys Gly Z Thr (Ala/Asn) Ala
(Val/Gly) (Gly/Ser/Lys) Lys Val (Gly/Ala/
Lys/Ile) (Leu/Ala) (SEQ ID:31)

15. <u>Tyr</u> Val Z Leu (Asn/Val/Leu) (Ser/Asn/Val) Z
Asn Leu Thr Asp (SEQ ID:32)

In the above related amino acid sequence patterns, the underlined amino acids are the reference amino acids $X_1$. The percentage of amino acids other than these reference amino acids was calculated, and amino acids having a high degree of frequency of contributing to each related amino acid sequence pattern, were extracted and simplified.

Thus, the following functional region-related amino acid sequence patterns were obtained:

1-1. <u>Cys</u> (Gly/Ser) Ala Z Gly Z (Gly/Ala)

(SEQ ID:33)

2-1. <u>His</u> Z Z Ser (Gly/Ser) Z Gly Z Gly Z Gly
(SEQ ID:34)

3-1. <u>Arg</u> Ser Z Z Z Ala Z Ser Z Z (Gly/Ala/Ser)
(SEQ ID:35)

4-1. <u>Ala</u> Z Ser Z Z Z Z Gly Z Z Gly Z Ala Z
(SEQ ID:36)

5-1. <u>Gly</u> (Gly/Ser) Z Z Z Ser Gly Ala Ser Gly
(Ala/Gly) Gly (Gly/Ser) (SEQ ID:37)

6-1. <u>Ser</u> (Gly/Ser) Ala Ser Z Gly Ser Z Gly Z Ser Z
Gly (SEQ ID:38)

7-1. <u>Thr</u> (Gly/Ala) Ala Z Ala (Ala/Ser) Z Ala
(Ala/Ser) (Gly/Ser) (SEQ ID:39)

8-1. <u>Asn</u> Z Z Ser (Gly/Ala) Ala Z (Ser/Ala) Gly
(Ser/Ala) Z Z Ser (SEQ ID:40)

and as non-functional region-related amino acid sequence patterns:

9-1. <u>Asp</u> Pro (Leu/Lys) Z Leu Z Z Val Lys Leu Leu
Leu (Gly/Lys) (SEQ ID:41)

10-1. <u>Glu</u> Z Z Z Z Lys Z Leu Leu (Asp/Leu) (Gly/Asp)
Z (Gly/Lys) (SEQ ID:42)

11-1. <u>Phe</u> (Gly/Leu) Asp Asp (Gly/Val) Z (Val/Asp)
Val Z Z (Gly/Leu) Val Gly Asp (SEQ ID:43)

12-1. <u>Lys</u> Leu (Asp/Lys) Z Z Gly Leu Gly (Asp/Lys)
(Val/Leu) Gly Leu Asp Leu (SEQ ID:44)

13-1. <u>Leu</u> Lys Asp Z (Gly/Asp) Z Z Z Leu (Asp/Lys)
(Asp/Val) (Asp/Lys) (Val/Leu) (Lys/Val)
(SEQ ID:45)

14-1. <u>Pro</u> Asp Lys Gly Z Z Z Z (Val/Gly) (Gly/Lys)
Lys Val (Gly/Lys) Leu (SEQ ID:46)

15-1. <u>Tyr</u> Val Z Leu (Val/Leu) Val Z Z Leu Z Asp
(SEQ ID:47)

In the above related amino acid sequence patterns, the underlined amino acids are reference amino acids $X_1$.

Amino acids belonging to $X_1$ and $X_2$ respectively were extracted from these related amino acid sequence patterns to obtain the following results.

Namely, there were obtained as supposed functional region-related patterns:

$X_1$ ... Cys, His, Arg, Ala, Gly, Ser, Thr, Asn

$X_2$ ... Gly, Ala, Ser

as supposed non-functional region-related patterns:

$X_1$ ... Asp, Glu, Phe, Lys, Leu, Pro, Tyr

$X_2$ ... Leu, Lys, Gly, Asp, Val

Then, the following results were obtained by investigation from the viewpoints described in the relation of sense and antisense.

Namely, for example, when an attention is given to Ala (alanine) as an $X_2$ in the supposed functional region-related patterns, one of the codons (although, usually, a codon means one having as a unit sequence consisting of three of 4 kinds of bases composing mRNA, this is conveniently applied to DNA in this specification) is "GCT". The antisense against this codon is CGA, and when this 3-base sequence is read in the direction of $5' \rightarrow 3'$, AGC is obtained whereby Ser (serine) is coded when read in the direction of $3' \rightarrow 5'$, "CGA" is Arg (arginine).

On the other hand, when GCT, a codon of Ala (alanine), is read in the reverse direction ($3' \rightarrow 5'$), a "TCG" is Ser (serine). All these amino acids (Ser and Arg) exist in amino acids of the group of $X_1$ in the patterns relating to the supposed functional region-related amino acid sequence patterns. On the other hand, when Lys (lysine) is taken as an example of $X_2$ in the supposed non-functional region-related patterns, similar relations occur, too. Namely, one of the codons of this amino acid is AAG. The antisense against this codon is TTC, and when this 3-base sequence is read in the direction of $5' \rightarrow 3'$, "CTT" is Leu (leucine). When read in the direction of $3' \rightarrow 5'$, "TTC" is Phe (phenylalanine).

On the other hand, when AAG, a codon of Lys (lysine), is read in the reverse direction ($3' \rightarrow 5'$), "GAA" is Glu (glutamic acid). All these amino acids (Leu, Phe and Glu) exist in amino acids of the groups of $X_1$ in the patterns relating to the supposed non-functional region-related amino acid sequence patterns. Therefore, it can be surmised that the amino acids of $X_1$ and $X_2$ are familiar with each other based on sense and antisense. Consequently, by utilizing this relation, it is possible to efficiently surmise an active site in a test polypeptide.

A trial to find specific active sites of a test polypeptide utilizing such relations was made previously in 1981 by J. Biro (Literature 11). He proposes a hypothesis that informational complementarity exists in intermolecular or intramolecular specific interaction regions of proteins. Intramolecular (in the same protein) active sites are explained exemplifying 9 kinds of polypeptides each composed of 115 or less amino acids.

However, it is concluded that in the same molecule, regions having informational complementarity extend all over the molecule, and the predominant of active sites in the said information is undistinguished from other regions. Therefore, it is difficult to specify the active sites from amino acid sequence of their precursors.

Thus, the present inventors at first suppose an amino acid sequence pattern represented by the following formula (II):

$$X_1' (Z)_m X_2' \qquad (II)$$

This amino acid sequence pattern is referred to as a supposed complementary amino acid sequence pattern for convenience sake. In the above formula (II), $X_1'$ and $X_2'$ are specific amino acids selected from 20 amino acids naturally occurring in proteins, and $X_1'$ and $X_2'$ are complementary with each other and related as above through their sense and antisense. In this sense, the above restriction requisite is essentially different from supposed amino acid sequences denoted by $X_1(Z)_n X_2$ [i.e., formula(I)]. $Z_s$ are the same or different unspecific amino acids, and m is 0 or an integer of 1 to about 30. However, since $X_1'$ and $X_2'$ are related as above, it is sufficient to use m of 0 or 1, i.e., $X_1' X_2'$ and $X_1' Z X_2'$. Thus, 160 kinds of supposed complementary amino acid sequence patterns were prepared for the formulae $X_1' X_2'$ and $X_1' Z X_2'$ ($X_1'$ and $X'_2$ are selected from 20 kinds of amino acids).

The present inventors further intensely investigated related and unrelated amino acid sequence patterns. Thus, 102 kinds of amino acid sequence patterns were prepared based on the repetitive sequences such as ab $(Z)_m$ ab and ab $(Z)_m$ ba (wherein a, b and Z are each any of 20 amino acids, and m is an integer of 0 to 15) and a substitution frequency constant disclosed in a literature (Literature 9), and the doublet sequences composed of the same or analogous amino acids (for example, Leu Leu, Leu Ile, His His, Trp Trp, Phe Trp, Pro Pro, Lys Lys, Arg Lys, etc.).

Then, these supposed complementary amino acid sequence patterns were applied to the amino acid sequences of the reference polypeptides, a multivariate analysis was carried out to induce a statistically stable discriminant function capable of distinguishing an active site or region from other. The thus obtained discriminant function can be applied to the amino acid sequence of the test polypeptide to surmise an active site or region in the amino acid sequence.

As the first specific example of the invention, the above 262 kinds of amino acid sequence patterns were applied to the amino acid sequences of 48 kinds of reference polypeptides denoted in Table 7 (the DNA sequences were quoted the description of BION/genbank database), and thereby a discriminant function was induced for surmising an active site or region (SAS, DISCRIM procedure, version 5 was used as an analytical method).

## Table 7

| | Protein name | Residue number | Active site[*] |
|---|---|---|---|
| 1 | Coagulation factor IX | 461 | 267, 315, 411 |
| 2 | Cathepsin G | 255 | 62, 106, 199 |
| 3 | Acetylcholinesterase | 649 | 276 |
| 4 | Cholinesterase | 602 | 226 |
| 5 | Cutinase | 228 | 99, 140, 208 |
| 6 | Esterase-6 | 548 | 209 |
| 7 | Aspartate protease | 380 | 84, 269 |
| 8 | Rhizopus pepsin | 352 | 60, 245 |
| 9 | Pepsinogen A | 385 | 91, 274 |
| 10 | Renin | 401 | 101, 286 |
| 11 | Collagenase | 469 | 219 |
| 12 | Enkephalinase | 749 | 584, 637 |
| 13 | Cell surface protease | 601 | 255 |
| 14 | Storomelysin | 477 | 219 |
| 15 | Thiol protease aleurain | 362 | 168, 308 |
| 16 | Calpain I larger (catalytic) subunit | 714 | 115, 272 |
| 17 | Cystein proteinase 1 | 343 | 142, 286 |
| 18 | Papain | 345 | 158, 292 |
| 19 | Endoglucanase EG1 | 256 | 149 |
| 20 | Lysosome α-glucosidase | 951 | 517 |
| 21 | Ricin | 565 | 201 |
| 22 | Sigma-like toxin I | 315 | 189 |
| 23 | Carboxypeptidase Y | 532 | 257, 508 |
| 24 | Penicillin-linked protein 6 | 400 | 66 |

| 25 | KEX 1 carboxypeptidase | 729 | 198, 470 |
| 26 | Micrococcus nuclease | 207 | 93, 101, 145 |
| 27 | Ribonuclease RH | 238 | 62, 125 |
| 28 | Cephalosporinase | 381 | 84 |
| 29 | ATP synthetase $\alpha$ chain | 513 | 373 |
| 30 | Fructose-1,6-biphosphatase | 338 | 275 |
| 31 | Alkaline phosphatase | 471 | 124 |
| 32 | Phosphate biphosphate aldolase | 364 | 364 |
| 33 | Arginosuccinate lyase | 464 | 51 |
| 34 | $\alpha$ enolase | 434 | 190, 412 |
| 35 | Ribulose biphosphate carboxylase | 487 | 204 |
| 36 | Chloramphenicol acetyltransferase | 213 | 189 |
| 37 | Galactose-1-phosphate uridyl transferase | 365 | 179, 181 |
| 38 | ADP-glucose synthetase | 431 | 39, 195 |
| 39 | Creatine kinase | 381 | 283 |
| 40 | Cytochrome C peroxidase | 362 | 116, 120, 243 |
| 41 | Malic acid dehydrogenase | 312 | 150, 177 |
| 42 | Aldehyde dehydrogenase | 497 | 298 |
| 43 | Glutamate dehydrogenase | 558 | 183 |
| 44 | DNA gyrase subunit A | 821 | 123 |
| 45 | Biphosphoglycerate mutase | 259 | 10, 61, 187 |
| 46 | DNA topoisomerase | 765 | 723 |
| 47 | Subtilisin BPN' | 382 | 139, 171, 328 |
| 48 | Urokinase | 431 | 224, 275, 376 |

The position of * was quoted from BION/swiss-prot database.

As a result, a discriminant function having a discrimination percentage of 89.2% (a priori probability of Near was 10.9%) was induced using 113 kinds of patterns out of 262 kinds of all the patterns. Further, the present inventors also conveniently induced discriminant functions having discrimination percentage of 88.6%, 85.7%

EP 0 494 502 A1

and 73.1% (a priori probabilities of Near were 20%, 30% and 50%, respectively) without changing the use of the patterns. Amino acid patterns preferentially used in these discriminant functions are shown below. Namely, there can be enumerated

```
      1. Gly Z Ser  2. Gly Thr  3. Val Z His
  4. Ala Z Cys  5. Ala Ser  6. Ser Z Arg  7. Leu Gln
  8. Val Tyr  9. Thr Z Typ  10. Thr Cys  11. Leu Z Asp
  12. Gly Z Thr  13. Leu Z Gln  14. Glu Phe  15. Glu Z Phe
  16. Arg Z Pro  17. Val Gln  18. Met Z His  19. Z₁Z₂
  ≧ 1.8  20. Ser Ser, etc.
```

In the above patterns, Z may be any of the unspecified 20 amino acids. Further, $Z_1$ and $Z_2$ are amino acid sequences sequenced in tandem in the amino acid sequences, and means a combination such that the total of their substitution frequency values is 1.8 or more. The substitution frequency values was based on Literature 9. It is apparent therein is that, among preferential amino acid patterns between 17 and 48 deferential amino acids used, the large majority of these preferential patterns are composed of the formula (II). Particularly, among them, patterns of Gly Z Ser, Gly Thr, Val Z His, Val Tyr, Leu Z Asp, Leu Z Gln, Val Gln and Ser Ser exhibit a high preferential order in any of the discriminant functions.

As the second specific example of the invention, the above 262 amino acid sequence patterns were applied to the amino acid sequences derived from the antisenses of 48 reference polypeptides to induce a discriminant function for surmising an active site or region (the analytical method was the same as above). In this case, since such proteins do not actually occur in nature, analysis for discriminating their active sites is carried out under the conditions of that the amino acid on the antisense corresponding to the amino acid at the position identified on the sense corresponds to the amino acid at the supposed active site.

Under the supposition, a discriminant function was deduced in the same way as above. As a result, a discrimination function was induced exhibiting a discrimination percentage of 89% (a priori probability of Near was 10.9%) using 95 patterns out of 262 kinds of patterns. Further, the present inventors also induced discriminant functions exhibiting discrimination percentage of 88.3%, 86.4% and 68.3% (a priori probabilities of Near were 20%, 30% and 50%, respectively) without changing the use of the patterns.

It is possible to apply the discriminant functions obtained by the first and second methods of the invention, individually and/or in combination, to every physiologically active polypeptide (or polynucleotide) whose amino acid sequence (or gene sequence) is known.

As exemplified in the following examples, according to the method of the invention, it is possible to efficiently surmise functional sites or regions of physiologically active polypeptides (or polynucleotides) whose amino acid sequences (or nucleic acid sequences) are known but whose active sites or regions are unknown, and thus the method is very useful for modification, improvement or the like of polypeptides (or polynucleotides).

The effectiveness of this invention is revealed below by examples.

Example 1

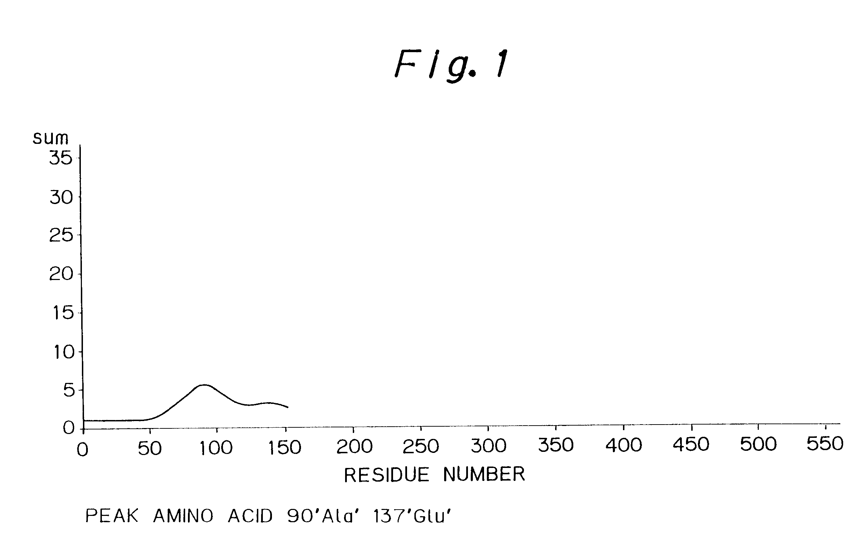

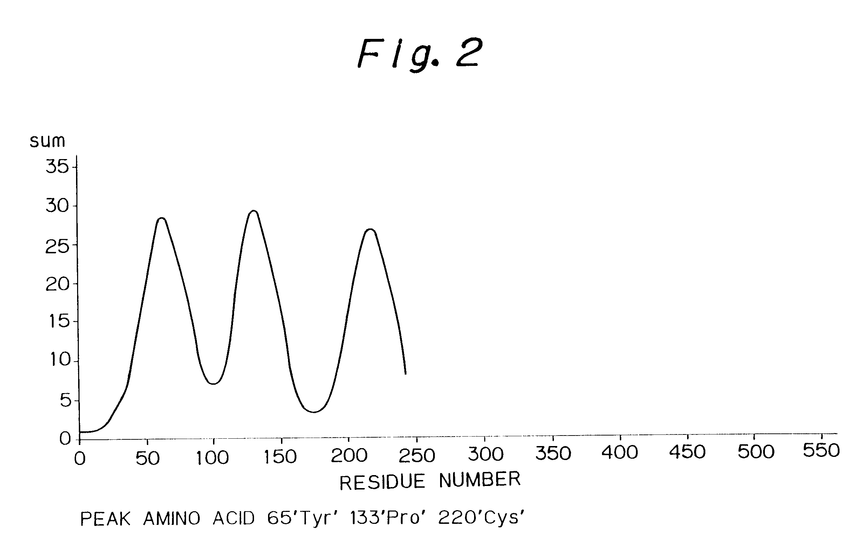

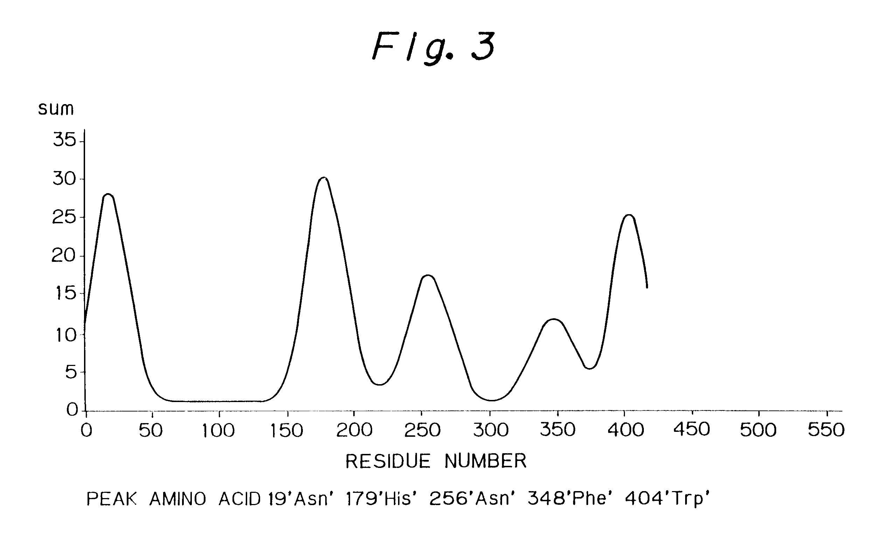

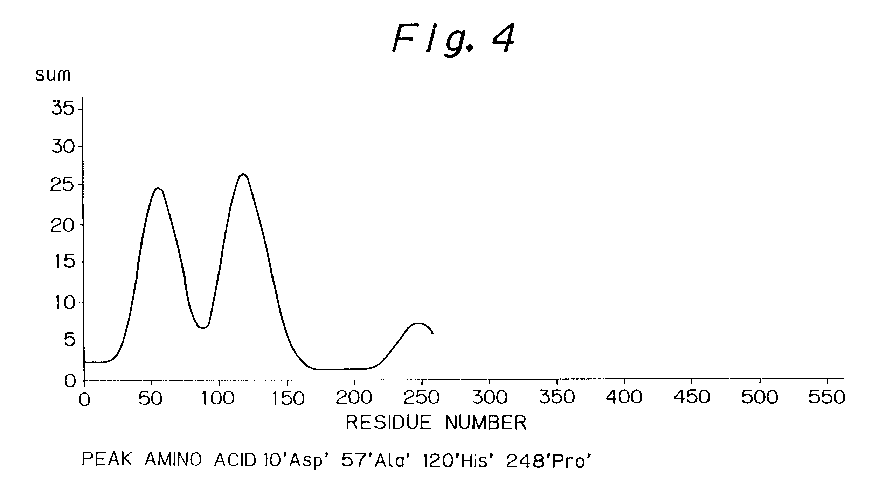

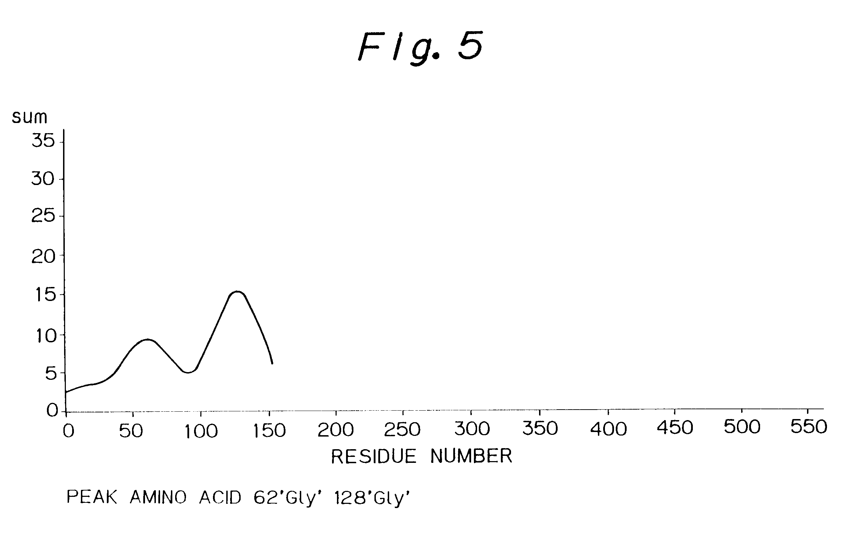

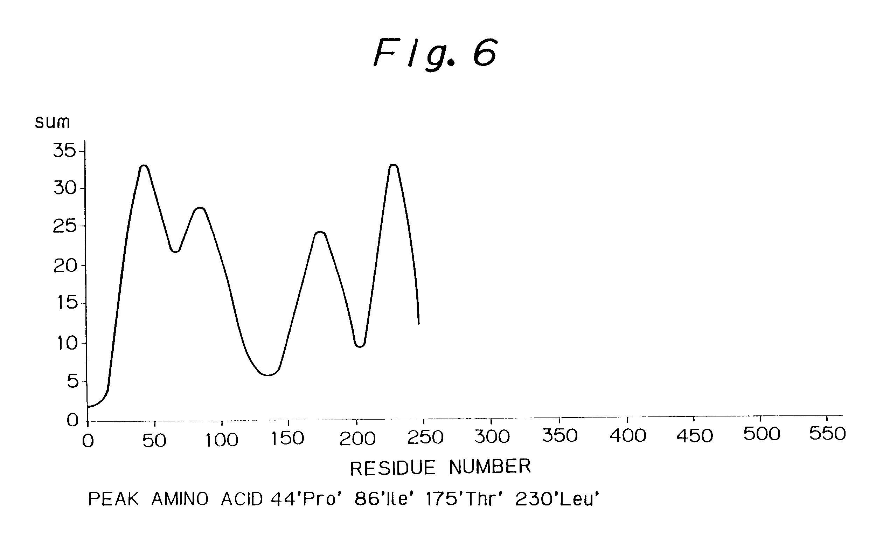

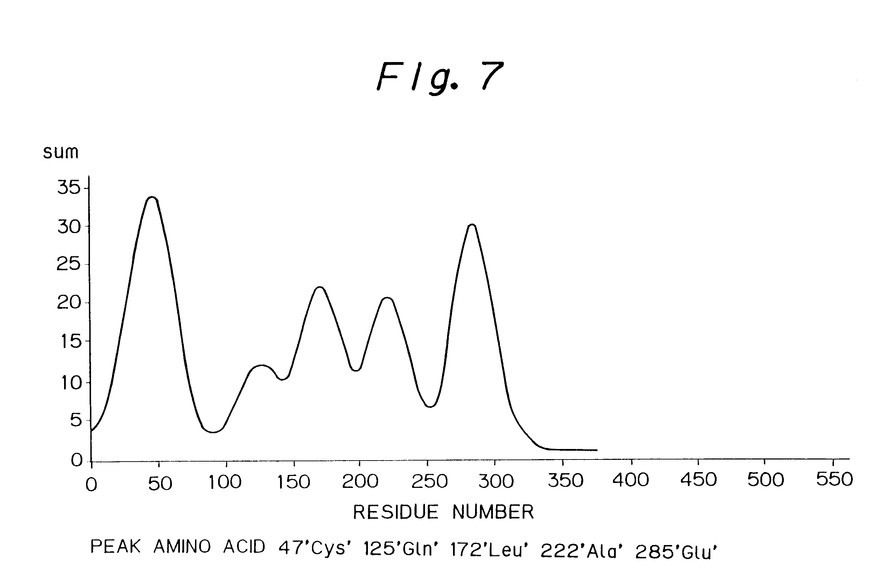

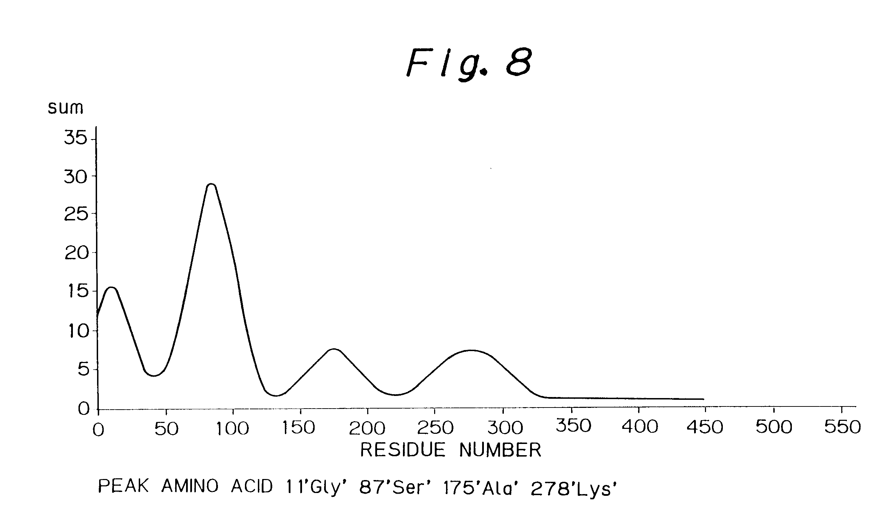

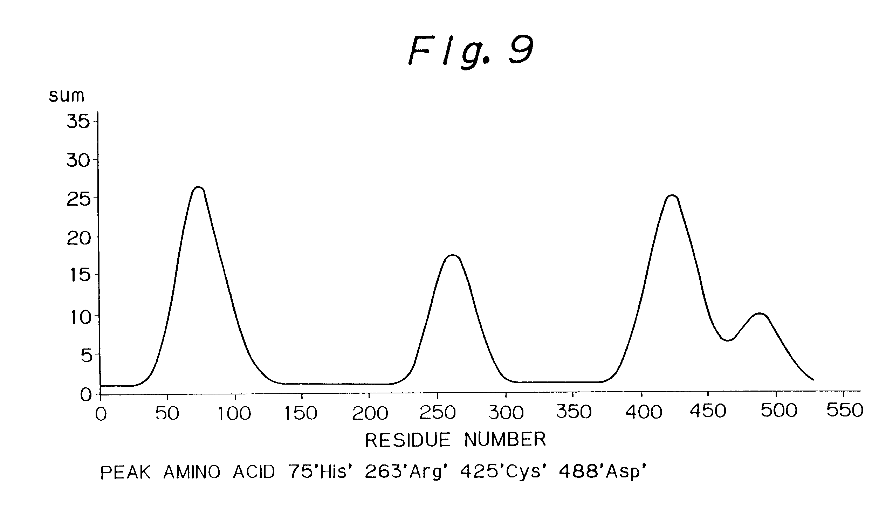

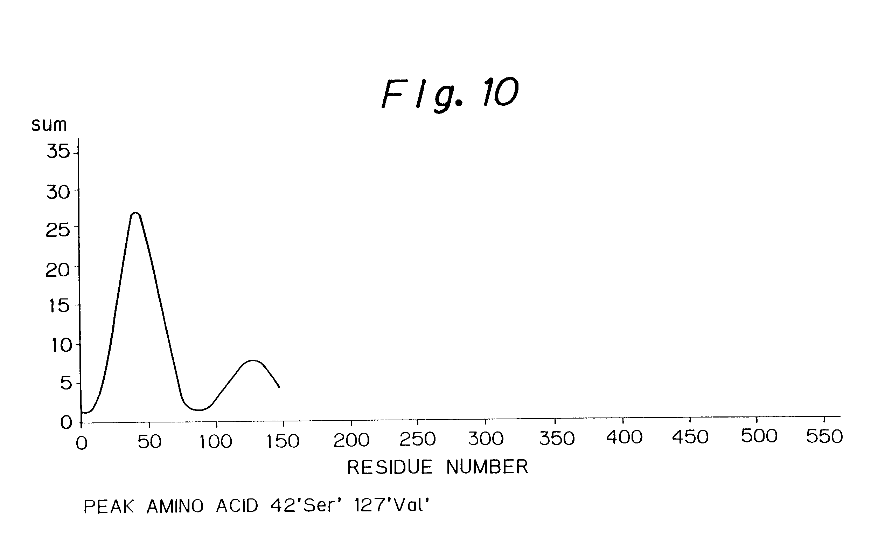

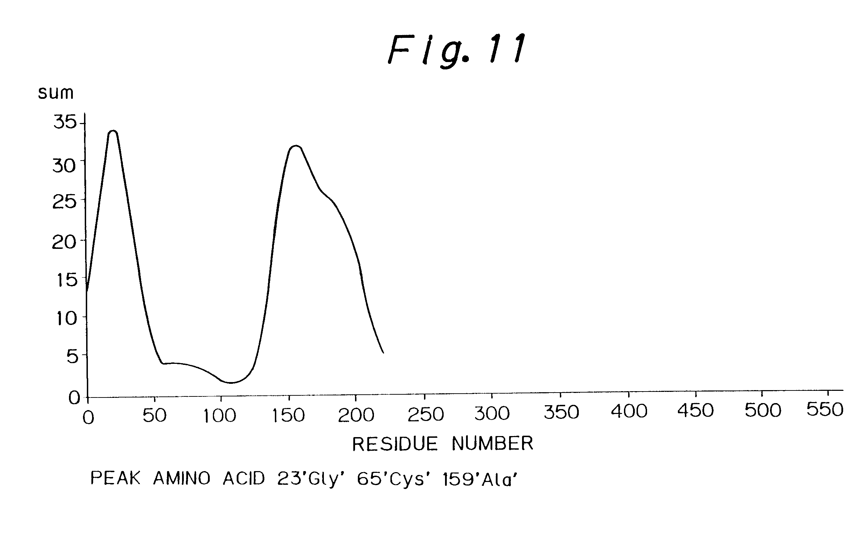

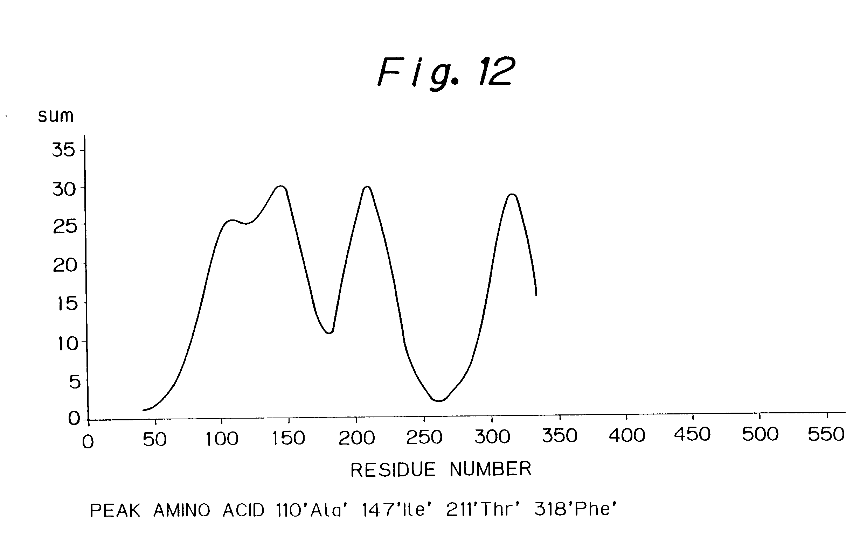

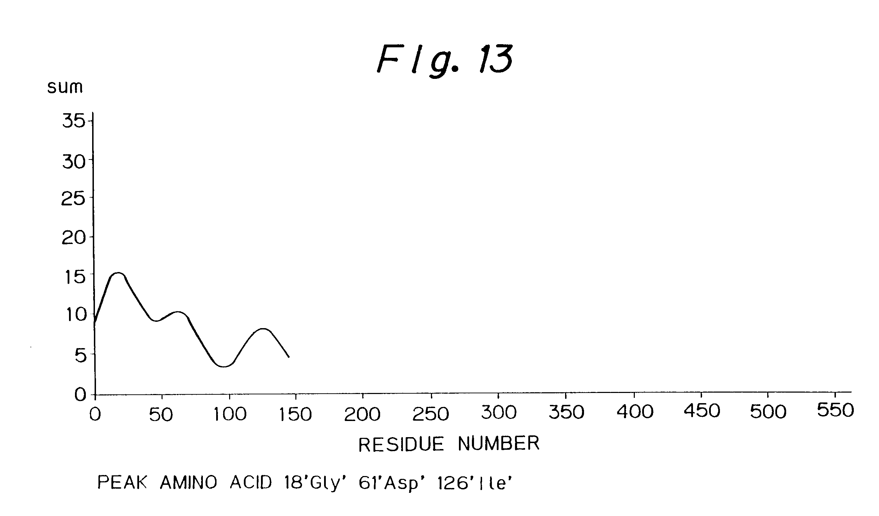

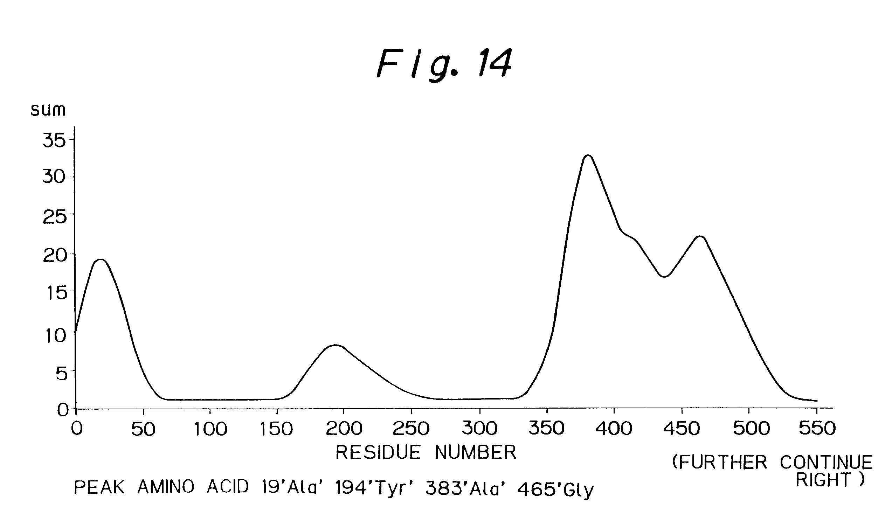

In order to verify the effectiveness of the method of the invention established as above, the method of the invention was applied to 14 enzymes whose active sites had been known (shown in Table 8, but they are the same as in Table 4 to Table 6), and the surmised results were compared with the actual active sites. The results are shown in Figures 1 to 14. In this connection, the active sites of these enzymes were determined based on homologous sequences to analogous enzymes (Table 1).

21

Table 8

|  | Protein name | Residue number | Action site |
|---|---|---|---|
| 1 | Ribonuclease (bovine) | 150 | 38, 67, 145 |
| 2 | Trypsinogen (canine) | 246 | 63, 107, 200 |
| 3 | Carboxypeptidase (rat) | 419 | 273, 358, 380 |
| 4 | Carbonic anhydrase I (human) | 261 | 107, 200 |
| 6 | Superoxide dismutase (human) | 154 | 64, 144 |
| 8 | Triosephosphate isomerase (chicken) | 248 | 165 |
| 9 | Alcohol dehydrogenase (human) | 375 | 49, 52 |
| 10 | Glutathione reductase (Escherichia coli) | 450 | 42, 47, 439 |
| 12 | Catalase (rat) | 527 | 75, 148 |
| 13 | Lysozyme C (bovine) | 147 | 53, 71 |
| 14 | Actinidine (kiwi fruit) | 220 | 25, 162 |
| 15 | Glyceraldehyde 3-phosphate dehydrogenase (human) | 335 | 152, 179 |
| 16 | Phospholipase A2 (bovine) | 145 | 70, 121 |
| 17 | Thermolysin (bacterium) | 552 | 379, 467 |

Example 2

The functional regions (namely, bidning sites, etc.) of proteins other than enzymes such as cytokines, hormones and receptors were verified using the above discriminant functions. The results are shown in Table 9 to Table 13.

Table 9

| | Protein | Number of amino acid | Membrane penetration region | Functional site | Surmised region | | |
|---|---|---|---|---|---|---|---|
| | | | | | 10N | 30N | 50N |
| 1. | PAI-1 | 402 | | 369-370 | 26 | 27,187,387 | 28,75,138,195,273,385 |
| 2. | Angiotensinogen | 484 | | 34-43 (angiotensin I) | 36,115,264 | 34,128,186,265, 308 | 33,125,194,266,335, 476 |
| 3. | CD4 | 458 | 397-418 | 53-69 | – | 248,287,385 | 38,130,236,290,387 |
| | | | | 68-78 | | | |
| | | | | 106-117 | | | |
| 4. | HIV envelope gp120 | 856 | | 413-456 | 37,361 | 30,51,223,350 | 27,70,105,188,229, 352,469,528,576,676, 806 |
| | | | | 419-427 | | 530,668,801 | |
| | | | | 301-324 (neutration epitope) | | | |
| 5. | Poliovirus capsid | 881 | | 671-684 (main antigen site) | 132,200,461, 638,800 | 138,202,287,385, 454,570,642,798 | 66,139,195,284,383, 460,573,649,791,844 |
| | | | | 737-746 (binding site) | | | |

EP 0 494 502 A1

Table 10

| | Protein | Number of amino acid | Membrane penetration region | Functional site | Surmised region | | |
|---|---|---|---|---|---|---|---|
| | | | | | 10N | 30N | 50N |
| 6. | Acetylcholine receptor α-subunit | 461 | 235-259, 267-285, 301-320, 433-451 | 152-166, 208-224 | 44 - | 44,143,206,326, 394,450 | 45,103,143,207,333, 395,444 |
| 7. | Interleukin 2 | 153 | | 56,58,60,62, 64,66 | | 128 | 10,100,138 |
| | | | | 35-39, 47-60 | | | |
| | | | | 37,40,142,144 | | | |
| 8. | Insulin receptor | 1382 | 957-979 | 47-147, 75-104 | 193,507,673, 762,866,1087 | 15,191,335,497, 565,692,754,865, 899,902,1022, 1087,1167,1232, 1346 | 23,122,190,244,333, 410,475,507,568,635, 693,753,862,911, 1027,1092,1169,1236, 1348 |
| | | | | 110-130 | | | |
| | | | | 232-333 | | | |
| | | | | 1029-1035,1057 (DNA binding site) | | | |

EP 0 494 502 A1

EP 0 494 502 A1

Table 11

| | Protein | Number of amino acid | Membrane penetration region | Functional site | Surmised region | | |
|---|---|---|---|---|---|---|---|
| | | | | | 10N | 30N | 50N |
| 9. | Insulin | 110 | | 25-54 (B chain) | – | 24 | 21 |
| | | | | 90-110 (A chain) | | | |
| | | | | 45-50 (binding site) | | | |
| 10. | Calcitonin (salmon I) | 136 | | 83-114 (calcitonin) | 120 | 121 | 21,72,119 |
| 11. | TNF-$\alpha$ | 233 | | 77-91 | 80 | 88 | 21,92,159,225 |
| 12. | $\beta$-amyloid (A4) | 695 | 625-648 | 597-638 ($\beta$-amiloid) | 141 | 142,457,609 | 37,102,148,194,363, 452,614,651 |
| 13. | Human-c-Ha-ras | 189 | | 12 | 21 | 17,125 | 16,78,130 |
| | | | | 172-189 (binding region) | | | |

EP 0 494 502 A1

Table 12

| | Protein | Number of amino acid | Membrane penetration region | Functional site | Surmised region | | |
|---|---|---|---|---|---|---|---|
| | | | | | 10N | 30N | 50N |
| 14. | il-receptor (tac) | 272 | 242-260 | 22-27,56-64 | - | 176 | 42,143,175,255 |
| 15. | HBsAg | 400 | | 21-47 | 151,368 | 31,146,211,364 | 31,69,149,213,291, 369 |
| 16. | hGH | 217 | | 38-45,80-99, 190-217 | - | 135 | 135,205 |
| 17. | mNGF | 307 | | 198(W),199(V) | 54,186 | 13,58,113,185, 252 | 15,58,120,188,214, 245 |
| 18. | IFN-γ | 166 | | 151-155, 156-166 | - | 38 | 39,147 |
| 19. | Estrogen receptor | 595 | | 353-362, 533-548 | 190,463,553 | 10,80,189,280, 397,467,549 | 11,81,189,246,283, 350,398,465,514,548 |
| 20. | Chorionic gonadotropin β-subunit | 165 | | 58-77 | - | 35 | 35,152 |

EP 0 494 502 A1

Table 13

| | Protein | Number of amino acid | Membrane penetration region | Functional site | Surmised region | | |
|---|---|---|---|---|---|---|---|
| | | | | | 10N | 30N | 50N |
| 21. | Glucagon | 179 | | 53-81 | - | 23 | 20,106,164 |
| 22. | ACTH (pig) | 267 | | 136-174(ACTH) | 85 | 86,153,235 | 19,77,150,231 |
| | | | | 217-224($\beta$-LPH) | | | |
| | | | | 227-267 ($\beta$-endorphin) | | | |
| 23. | Human c-fos | 380 | | 143-158 (basic motif) | 108 | 35,105,302 | 34,104,161,225,305, 357 |
| | | | | 165-193 (leucine zipper) | | | |
| 24. | TGF-$\alpha$ | 160 | | 73-82 | 26,85 | 23,79,146 | 22,74,146 |
| 25. | ANP | 152 | | 123-150(ANP) | - | 33,75 | 21,73,124 |
| 26. | Somatostatin | 116 | | 89-116 (somatostatin) | 104 | 28,104 | 27,104 |

Example 3

As a typical example in substances other than proteins, a surmised result is denoted on the catalytically active site of a tobacco ringspot virus (TRSV), one of ribozymes. It is known that TRSV is an RNA composed of 359 nucleotides and its catalytically active site exists in the 50 nucleotides between the thymine nucleotide at the 175th position and the thymine nucleotide at the 224th position. The amino acid sequence obtained by translation of this nucleotide sequence is as follows.

```
Thr Gly Cys Ala Phe Arg Ser Asp Glu Ser Val Arg Thr
Lys Gln Asp Cys Gln Val Ala Glu Ser His His Val Asn
· · Thr Val Leu Arg Ser Val Gly Val Cys Tyr Leu Val
Gly Gly Gly Asp Cys Ser Leu Arg Val Gly Ala Ala Val
· Leu Val Lys Ala Tyr Gln Val Ile Tyr His Asn Val
Cys Phe Ser Gly · Leu Leu Cys Leu Leu Cys His Trp
Phe Pro Asp Leu Ala Leu Ala Ala Thr Gly Tyr Ser His
Ser Thr Trp Lys Phe Glu Arg Pro Arg Leu Tyr Thr Met
Arg Gly Glu Ser Lys Leu Phe · Pro Asp Thr Leu
(SEQ ID:48)
```

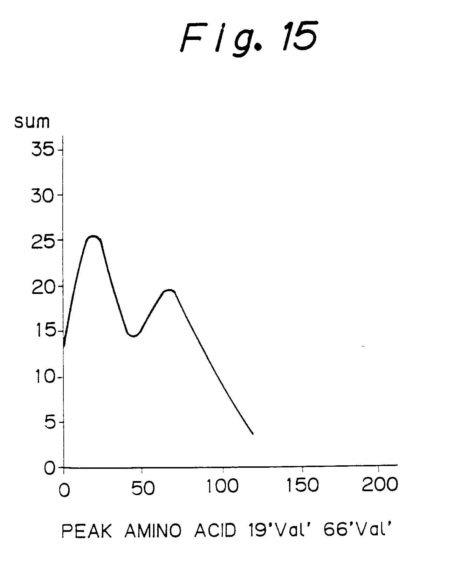

The result obtained by applying the above discriminant function to this sequence is shown in Fig. 15.

As mentioned above, according to the method of the invention, it is possible to effectively surmise the active site of a physiologically active polypeptide whose amino acid sequence is known but whose active site is not known, and thus the method is very useful tool for uderstanding the functional mechanism of polypeptides, modification and improvement of polypeptides, etc.

Literatures

1. A. E. Cabrielian, V. S. Ivanov and A. T. Kozhich, CABIOS, 6, 1 (1990).

2. T. C. Hodgman CABIOS, 2, 181 (1986): idem, ibidem, 5, 1 (1989).

3. T. P. Hopp and K. R. Woods, Proc. Natl. Acad.

Sci., <u>78</u>, 3824 (1981).

J. Kyte and R. F. Doolittle, J. Mol. Biol., <u>157</u>, 105 (1982).

4. K. L. Bost, E. M. Smith and J. E. Blalock, Biochem. Biophys. Res. Commun., <u>128</u>, 1373 (1985).

5. R. F. Doolittle D. F. Feng and M. S. Johnson, Nature, <u>307</u>, 558 (1984).

6. C. D. DeLisi and J. A. Berzofsky, Proc. Natl. Acad. Sci., <u>82</u>, 7048 (1985).

7. M. J. J. M. Zvclevil and M. J. E. Sternberg, Protein Engineering, <u>2</u>, 127 (1988).

8. F. Vondcrvist and I. Simon, Biochem. Biophys. Res. Commun., <u>139</u>, 11 (1986).

9. M. O. Dayhoff. Atlas of protein sequence and structure. Natl. Biom. Res. Foundation, Washington, D. C. (1972).

10. M. Ballivet and S. A. Kauffman, WO 86/05803 (1985).

11. J. Biro, Medical Hypotheses, <u>7</u>, 969 (1981), ibid. <u>7</u>, 981 (1981), ibid. <u>7</u>, 995 (1981).

12. Y. Seto, Y. Ikeuchi and M. Kanehisa, Proteins: Structure, Function and Genetics, <u>8</u>, 341 (1990).

## SEQUENCE LISTING

SEQ ID NO:  1

    (i)   SEQUENCE CHARACTERISTICS:

        (A)  LENGTH:  7 amino acids

        (B)  TYPE:  amino acid

        (D)  TOPOLOGY:  linear

   (ii)  MOLECULE TYPE:  peptide

  (iii)  HYPOTHETICAL:  yes

    (ix)  FERTURE:

        (D)  OTHER INFORMATION:

           Xaa2:  Gly or Ser;  Xaa7:  Gly or Ala;

           Xaa4 and Xaa6:  any amino acid

    (xi)  SEQUENCE DESCRIPTION:  SEQ ID NO:  1

      Cys Xaa Ala Xaa Gly Xaa Xaa
         1           5

SEQ ID NO:  2

    (i)   SEQUENCE CHARACTERISTICS:

        (A)  LENGTH:  11 amino acids

        (B)  TYPE:  amino acid

        (D)  TOPOLOGY:  linear

   (ii)  MOLECULE TYPE:  peptide

  (iii)  HYPOTHETICAL:  yes

    (ix)  FERTURE

        (D)  OTHER INFORMATION:

           Xaa2, Xaa3, Xaa6, Xaa8, Xaa9:  any amino

           acid;  Xaa4:  Gly or Ser

(xi)  SEQUENCE DESCRIPTION:  SEQ ID NO:  2

His Xaa Xaa Ser Xaa Xaa Gly Xaa Gly Xaa Gly
1                    5                    10

SEQ ID NO:  3

(i)  SEQUENCE CHARACTERISTICS:

(A)  LENGTH:  11 amino acids

(B)  TYPE:  amino acid

(D)  TOPOLOGY:  linear

(ii)  MOLECULE TYPE:  peptide

(iii)  HYPOTHETICAL:  yes

(ix)  FERTURE

(D)  OTHER INFORMATION:

Xaa3, Xaa4, Xaa5, Xaa7, Xaa9, Xaa10:  any

amino acid;  Xaa11:  Gly, Ala or Ser

(xi)  SEQUENCE DESCRIPTION:  SEQ ID NO:  3

Arg Ser Xaa Xaa Xaa Ala Xaa Ser Xaa Xaa Xaa
1                    5                    10

SEQ ID NO:  4

(i)  SEQUENCE CHARACTERISTICS:

(A)  LENGTH:  14 amino acids

(B)  TYPE:  amino acid

(D)  TOPOLOGY:  linear

(ii)  MOLECULE TYPE:  peptide

(iii)  HYPOTHETICAL:  yes

(ix)  FERTURE

(D)  OTHER INFORMATION:

All Xaa:  any amino acid

(xi) SEQUENCE DESCRIPTION: SEQ ID NO: 4

Ala Xaa Ser Xaa Xaa Xaa Xaa Gly Xaa Xaa Gly Xaa
1                   5                   10
Ala Xaa

SEQ ID NO: 5

(i) SEQUENCE CHARACTERISTICS:

(A) LENGTH: 13 amino acids

(B) TYPE: amino acid

(D) TOPOLOGY: linear

(ii) MOLECULE TYPE: peptide

(iii) HYPOTHETICAL: yes

(ix) FERTURE

(D) OTHER INFORMATION:

Xaa3, Xaa4, Xaa5: any amino acid; Xaa:

Gly or Ser; Xaa11: Ala or Gly; Xaa13:

Gly or Ser

(xi) SEQUENCE DESCRIPTION: SEQ ID NO: 5

Gly Xaa Xaa Xaa Xaa Ser Gly Ala Ser Gly Xaa Gly
1                   5                   10
Xaa

SEQ ID NO: 6

(i) SEQUENCE CHARACTERISTICS:

(A) LENGTH: 13 amino acids

(B) TYPE: amino acid

(D) TOPOLOGY: linear

(ii) MOLECULE TYPE: peptide

(iii) HYPOTHETICAL: yes

(ix) FERTURE

(D)  OTHER INFORMATION:

        Xaa5, Xaa8, Xaa10, Xaa12:  any amino acid;

        Xaa2:  Gly or Ser

(xi)  SEQUENCE DESCRIPTION:  SEQ ID NO:  6

        Ser Xaa Ala Ser Xaa Gly Ser Xaa Gly Xaa Ser Xaa
         1                5                 10
        Gly

SEQ ID NO:  7

  (i)  SEQUENCE CHARACTERISTICS:

    (A)  LENGTH:  10 amino acids

    (B)  TYPE:  amino acid

    (D)  TOPOLOGY:  linear

 (ii)  MOLECULE TYPE:  peptide

(iii)  HYPOTHETICAL:  yes

 (ix)  FERTURE

    (D)  OTHER INFORMATION:

        Xaa4, Xaa7:  any amino acid;  Xaa2:  Gly

        or Ala;  Xaa6:  Ala or Ser;  Xaa10:  Ala

        or Ser;  Xaa10:  Gly or Ser

(xi)  SEQUENCE DESCRIPTION:  SEQ ID NO:  7

        Thr Xaa Ala Xaa Ala Xaa Xaa Ala Xaa Xaa
         1               5                 10

SEQ ID NO:  8

  (i)  SEQUENCE CHARACTERISTICS:

    (A)  LENGTH:  13 amino acids

    (B)  TYPE:  amino acid

    (D)  TOPOLOGY:  linear

 (ii)  MOLECULE TYPE:  peptide

(iii)  HYPOTHETICAL:  yes

(ix)  FERTURE

(D)  OTHER INFORMATION:

Xaa2, Xaa3, Xaa7, Xaal1, Xaal2:  any amino

acid;  Xaa5:  Gly or Ala;  Xaa8:  Ser or

Ala;  Xaal0:  Ser or Ala

(xi)  SEQUENCE DESCRIPTION:  SEQ ID NO:  8

```
Asn Xaa Xaa Ser Xaa Ala Xaa Xaa Gly Xaa Xaa Xaa
 1               5                   10
Ser
```

SEQ ID NO:  9

(i)  SEQUENCE CHARACTERISTICS:

(A)  LENGTH:  13 amino acids

(B)  TYPE:  amino acid

(D)  TOPOLOGY:  linear

(ii)  MOLECULE TYPE:  peptide

(iii)  HYPOTHETICAL:  yes

(ix)  FERTURE

(D)  OTHER INFORMATION:

Xaa4, Xaa6, Xaa7:  any amino acid;  Xaa3:

Leu or Lys;  Xaal3:  Gly or Lys

(xi)  SEQUENCE DESCRIPTION:  SEQ ID NO:  9

```
Asp Pro Xaa Xaa Leu Xaa Xaa Val Lys Leu Leu Leu
 1               5                   10
Xaa
```

SEQ ID NO:  10

(i)  SEQUENCE CHARACTERISTICS:

(A)  LENGTH:  13 amino acids

          (B)   TYPE:   amino acid

          (D)   TOPOLOGY:   linear

    (ii)   MOLECULE TYPE:   peptide

   (iii)   HYPOTHETICAL:   yes

    (ix)   FERTURE

          (D)   OTHER INFORMATION:

                Xaa2, Xaa3, Xaa4, Xaa5, Xaa7, Xaal2:   any

                amino acid;   Xaal0:   Asp or Leu;   Xaall:

                Gly or Asp;   Xaal3:   Gly or Lys

    (xi)   SEQUENCE DESCRIPTION:   SEQ ID NO:   10

          Glu Xaa Xaa Xaa Xaa Lys Xaa Leu Leu Xaa Xaa Xaa
            1                   5                   10
          Xaa

SEQ ID NO:   11

     (i)   SEQUENCE CHARACTERISTICS:

          (A)   LENGTH:   14 amino acids

          (B)   TYPE:   amino acid

          (D)   TOPOLOGY:   linear

    (ii)   MOLECULE TYPE:   peptide

   (iii)   HYPOTHETICAL:   yes

    (ix)   FERTURE

          (D)   OTHER INFORMATION:

                Xaa6, Xaa9, Xaal0:   any amino acid;   Xaa2:

                Gly or Leu;   Xaa5:   Gly or Val;   Xaa7:   Val

                or Asp;   Xaall:   Gly or Leu

    (xi)   SEQUENCE DESCRIPTION:   SEQ ID NO:   11

          Phe Xaa Asp Asp Xaa Xaa Xaa Val Xaa Xaa Xaa Val
            1                   5                   10

Gly Asp

SEQ ID NO: 12

    (i)  SEQUENCE CHARACTERISTICS:

        (A)  LENGTH:  14 amino acids

        (B)  TYPE:  amino acid

        (D)  TOPOLOGY:  linear

   (ii)  MOLECULE TYPE:  peptide

  (iii)  HYPOTHETICAL:  yes

   (ix)  FERTURE

        (D)  OTHER INFORMATION:

           Xaa4, Xaa5:  any amino acid;  Xaa3:  Asp

           or Lys;  Xaa9:  Asp or Lys;  Xaa10:  Lal

           or Leu

   (xi)  SEQUENCE DESCRIPTION:  SEQ ID NO:  12

        Lys Leu Xaa Xaa Xaa Gly Leu Gly Xaa Xaa Gly Leu
        1           5          10
        Asp Leu

SEQ ID NO: 13

    (i)  SEQUENCE CHARACTERISTICS:

        (A)  LENGTH:  14 amino acids

        (B)  TYPE:  amino acid

        (D)  TOPOLOGY:  linear

   (ii)  MOLECULE TYPE:  peptide

  (iii)  HYPOTHETICAL:  yes

   (ix)  FERTURE

        (D)  OTHER INFORMATION:

           Xaa4, Xaa6, Xaa7, Xaa8:  any amino acid;

Xaa5: Gly or Asp; Xaa10: Asp or Lys;

Xaa11: Asp or Val; Xaa12: Asp or Lys;

Xaa13: Val or Leu; Xaa14: Lys or Val

(xi) SEQUENCE DESCRIPTION: SEQ ID NO: 13

Leu Lys Asp Xaa Xaa Xaa Xaa Xaa Leu Xaa Xaa Xaa
1                   5                        10
Xaa Xaa

SEQ ID NO: 14

(i) SEQUENCE CHARACTERISTICS:

(A) LENGTH: 14 amino acids

(B) TYPE: amino acid

(D) TOPOLOGY: linear

(ii) MOLECULE TYPE: peptide

(iii) HYPOTHETICAL: yes

(ix) FERTURE

(D) OTHER INFORMATION:

Xaa5, Xaa6, Xaa7, Xaa8: any amino acid;

Xaa9: Val or Gly; Xaa10: Gly or Lys;

Xaa 13: Gly or Lys

(xi) SEQUENCE DESCRIPTION: SEQ ID NO: 14

Pro Asp Lys Gly Xaa Xaa Xaa Xaa Xaa Xaa Lys Val
1                   5                        10
Xaa Leu

SEQ ID NO: 15

(i) SEQUENCE CHARACTERISTICS:

(A) LENGTH: 11 amino acids

(B) TYPE: amino acid

(D) TOPOLOGY: linear

(ii)   MOLECULE TYPE:  peptide

(iii)  HYPOTHETICAL:  yes

(ix)   FERTURE

(D)   OTHER INFORMATION:

Xaa3, Xaa7, Xaa8 Xaa10:  any amino acid;

Xaa5:  Vol or Leu

(xi)   SEQUENCE DESCRIPTION:  SEQ ID NO:  15

Tyr Val Xaa Leu Xaa Val Xaa Xaa Leu Xaa Asp
1               5                   10

SEQ ID NO:  16

(i)   SEQUENCE CHARACTERISTICS:

(A)   LENGTH:  7 amino acids

(B)   TYPE:  amino acid

(D)   TOPOLOGY:  linear

(ii)   MOLECULE TYPE:  peptide

(iii)  HYPOTHETICAL:  yes

(ix)   FERTURE

(D)   OTHER INFORMATION:

Xaa4, Xaa6: any amino acid;  Xaa2:  Gly or

Ser;  Xaa7:  Gly or Ala

(xi)   SEQUENCE DESCRIPTION:  SEQ ID NO:  16

Cys Xaa Ala Xaa Gly Xaa Xaa
1               5

SEQ ID NO:  17

(i)   SEQUENCE CHARACTERISTICS:

(A)   LENGTH:  13 amino acids

(B)   TYPE:  amino acid

```
        (D)  TOPOLOGY:  linear

  (ii)  MOLECULE TYPE:  peptide

 (iii)  HYPOTHETICAL:  yes

  (ix)  FERTURE

        (D)  OTHER INFORMATION:

             Xaa2, Xaa4 Xaa10, Xaa 12: any amino acid;

             Xaa1:  Gly or Ala;  Xaa6:  Gly or Ser;

             Xaa8:  Gly or Ser;  Xaa13:  Gly or Ala

  (xi)  SEQUENCE DESCRIPTION:  SEQ ID NO:  17

        Xaa Xaa Gly Xaa Ala Xaa Cys Xaa Ala Xaa Gly Xaa
          1               5                    10
        Xaa

SEQ ID NO:  18

    (i)  SEQUENCE CHARACTERISTICS:

        (A)  LENGTH:  7 amino acids

        (B)  TYPE:  amino acid

        (D)  TOPOLOGY:  linear

  (ii)  MOLECULE TYPE:  peptide

 (iii)  HYPOTHETICAL:  yes

  (ix)  FERTURE

        (D)  OTHER INFORMATION:

             Xaa4, Xaa6: any amino acid;  Xaa2:  Gly or

             Ser;  Xaa5:  Gly or Arg;  Xaa7:  Gly or

             Ala

  (xi)  SEQUENCE DESCRIPTION:  SEQ ID NO:  18

        Cys Xaa Ala Xaa Xaa Xaa Xaa
          1               5

SEQ ID NO:  19
```

(i)   SEQUENCE CHARACTERISTICS:

    (A)   LENGTH:   11 amino acids

    (B)   TYPE:   amino acid

    (D)   TOPOLOGY:   linear

(ii)   MOLECULE TYPE:   peptide

(iii)   HYPOTHETICAL:   yes

(ix)   FERTURE

    (D)   OTHER INFORMATION:

        Xaa2, Xaa3, Xaa6, Xaa8, Xaa10: any amino

        acid;   Xaa5:   Gly or Ser

(xi)   SEQUENCE DESCRIPTION:   SEQ ID NO:   19

His Xaa Xaa Ser Xaa Xaa Gly Xaa Gly Xaa Gly
   1             5               10

SEQ ID NO:   20

(i)   SEQUENCE CHARACTERISTICS:

    (A)   LENGTH:   12 amino acids

    (B)   TYPE:   amino acid

    (D)   TOPOLOGY:   linear

(ii)   MOLECULE TYPE:   peptide

(iii)   HYPOTHETICAL:   yes

(ix)   FERTURE

    (D)   OTHER INFORMATION:

        Xaa3, Xaa4, Xaa7, Xaa9, Xaa10: any amino

        acid;  Xaa11:  Gly, Ala or Ser;   Xaa12:

        Ser or Val

(xi)   SEQUENCE DESCRIPTION:   SEQ ID NO:   20

```
          Arg Ser Xaa Xaa Cys Ala Xaa Ser Xaa Xaa Xaa Xaa
           1               5                   10
```

SEQ ID NO:  21

    (i)    SEQUENCE CHARACTERISTICS:

        (A)   LENGTH:  13 amino acids

        (B)   TYPE:  amino acid

        (D)   TOPOLOGY:  linear

   (ii)   MOLECULE TYPE:  peptide

 (iii)   HYPOTHETICAL:  yes

   (ix)   FERTURE

        (D)   OTHER INFORMATION:

           Xaa3:  any amino acid;  Xaa2:  Ser, Thr,

           Val or Cys;  Xaa4:  Thr, Leu or Asn;

           Xaa5:  Thr or Asn;  Xaa7:  Gly, Thr or

           Asn;  Xaa8:  Thr or Glu;  Xaa9:  Ile or

           Asn;  Xaa10:  Gly or Arg;  Xaa11:  Glu or

           Glum

   (xi)   SEQUENCE DESCRIPTION:  SEQ ID NO:  21

```
     Ala Thr Xaa Xaa Xaa Xaa Cys Xaa Xaa Xaa Xaa Xaa
      1               5                   10
     Ala
```

SEQ ID NO:  22

    (i)    SEQUENCE CHARACTERISTICS:

        (A)   LENGTH:  14 amino acids

        (B)   TYPE:  amino acid

        (D)   TOPOLOGY:  linear

   (ii)   MOLECULE TYPE:  peptide

 (iii)   HYPOTHETICAL:  yes

        (ix)  FERTURE

            (D)  OTHER INFORMATION:

                Xaa2:  Gly, Ser or Thr;  Xaa5:  Asn, His

                or Cys;  Xaa6:  Ser, Pro or Phe;  Xaa7:

                Gly, His or Cys;  Xaa8:  Ile or Asn;

                Xaa9:  Gly or Thr;  Xaa10:  Ala, Val, Gly,

                Arg, His, Tyr;  Xaa12:  Gly or Ser

        (xi)  SEQUENCE DESCRIPTION:  SEQ ID NO:  22

        Gly Xaa Gln Tyr Xaa Xaa Xaa Ala Xaa Xaa Xaa Gly
        1                   5                   10
        Xaa Val

SEQ ID NO:  23

        (i)  SEQUENCE CHARACTERISTICS:

            (A)  LENGTH:  14 amino acids

            (B)  TYPE:  amino acid

            (D)  TOPOLOGY:  linear

        (ii)  MOLECULE TYPE:  peptide

        (iii)  HYPOTHETICAL:  yes

        (ix)  FERTURE

            (D)  OTHER INFORMATION:

                Xaa2:  Gly, Lys, Glu, Cys, Ser or Arg;

                Xaa4:  Ser, Leu, Asn or His;  Xaa5:  Glu,

                Val, His or Phe;  Xaa6:  Gly, Thr or Val;

                Xaa7:  Lys, Ser or Tyr;  Xaa8:  Asn, Gln

                or Arg;  Xaa9:  Gly or Thr;  Xaa10:  Asn,

                Ile or Glu;  Xaa11:  Gln, Ile, Ser or Arg;

                Xaa13:  Asn or Gly;  Xan14:  Ile or Glu

(xi) SEQUENCE DESCRIPTION: SEQ ID NO: 23

Ser Xaa Ala Xaa Xaa Xaa Xaa Xaa Xaa Xaa Xaa Arg
1               5                   10
Xaa Xaa

SEQ ID NO: 24

    (i) SEQUENCE CHARACTERISTICS:

        (A) LENGTH: 13 amino acids

        (B) TYPE: amino acid

        (D) TOPOLOGY: linear

   (ii) MOLECULE TYPE: peptide

  (iii) HYPOTHETICAL: yes

   (ix) FERTURE

        (D) OTHER INFORMATION:

           Xaa2: Gly or Ala;  Xaa3: Ala or Ile;

           Xaa5: Ala, Val or Leu;  Xaa6: Ala, Ser

           or Leu;  Xaa7: Asp, Pro or Glu;  Xaa8:

           Ala, Thr or Leu;  Xaa9: Ala, Ser or Phe;

           Xaa10: Gly, Ser or Val;  Xaa11: Asp, Leu

           or Val;  Xaa12: Asp, Lys or Ile;  Xaa13:

           Val or Leu

    (xi) SEQUENCE DESCRIPTION: SEQ ID NO: 24

Thr Xaa Xaa Thr Xaa Xaa Xaa Xaa Xaa Xaa Xaa Xaa
1               5                   10
Xaa

SEQ ID NO: 25

    (i) SEQUENCE CHARACTERISTICS:

        (A) LENGTH: 13 amino acids

        (B) TYPE: amino acid

          (D)  TOPOLOGY:  linear

     (ii)  MOLECULE TYPE:  peptide

     (iii)  HYPOTHETICAL:  yes

     (ix)  FERTURE

          (D)  OTHER INFORMATION:

               Xaa2:  Thr or Leu;  Xaa4:  Val or Ser;

               Xaa5:  Gly or Ala;  Xaa7:  Lys or Ile;

               Xaa8:  Ser, Ala or Lys;  Xaa10:  Ser, Asp

               or Alu;  Xaa13:  Ser or Lys;  Xaa11,

               Xaa12:  any amino acid

     (xi)  SEQUENCE DESCRIPTION:  SEQ ID NO:  25

          Asn Xaa Leu Xaa Xaa Ala Xaa Xaa Gly Xaa Xaa Xaa
           1               5                   10
          Xaa

SEQ ID NO:  26

     (i)  SEQUENCE CHARACTERISTICS:

          (A)  LENGTH:  14 amino acids

          (B)  TYPE:  amino acid

          (D)  TOPOLOGY:  linear

     (ii)  MOLECULE TYPE:  peptide

     (iii)  HYPOTHETICAL:  yes

     (ix)  FERTURE

          (D)  OTHER INFORMATION:

               Xaa3:  Ala, Leu or Lys;  Xaa4:  Ile or

               Phe;  Xaa10:  Leu or Glu;  Xaa11:  Ser,

               Leu or Glu;  Xaa12:  Gly or Leu

     (xi)  SEQUENCE DESCRIPTION:  SEQ ID NO:  26

```
Asp Pro Xaa Xaa Leu Ala Phe Val Lys Xaa Xaa Xaa
 1                   5                   10
Lys Phe
```

SEQ ID NO: 27

  (i)   SEQUENCE CHARACTERISTICS:

     (A)   LENGTH:  13 amino acids

     (B)   TYPE:  amino acid

     (D)   TOPOLOGY:  linear

  (ii)  MOLECULE TYPE:  peptide

 (iii)  HYPOTHETICAL:  yes

  (ix)  FERTURE

     (D)   OTHER INFORMATION:

        Xaa2, Xaa4, Xaa5, Xaa7:  any amino acid;

        Xaa6:  Lys, Phe or Ser;  Xaa8:  Pro or

        Leu;  Xaa10:  Asp, Leu or Ile;  Xaa11:

        Gly or Asp;  Xaa12:  Ser or Ile;  Xaa13:

        Gly, Lys or Phe

  (xi)  SEQUENCE DESCRIPTION:  SEQ ID NO:  27

```
Glu Xaa Ile Xaa Xaa Xaa Xaa Xaa Leu Xaa Xaa Xaa
 1               5                   10
Xaa
```

SEQ ID NO: 28

  (i)   SEQUENCE CHARACTERISTICS:

     (A)   LENGTH:  14 amino acids

     (B)   TYPE:  amino acid

     (D)   TOPOLOGY:  linear

  (ii)  MOLECULE TYPE:  peptide

 (iii)  HYPOTHETICAL:  yes

(ix)  FERTURE

(D)  OTHER INFORMATION:

Xaa2:  Gly or Leu;  Xaa3:  Asp or Ser;

Xaa4:  Ile or Asp;  Xaa5:  Gly or Val;

Xaa6:  Ser or Glu;  Xaa7:  Val or Asp;

Xaa10:  any amino acid;  Xaa11:  Gly or

Leu;  Xaa12:  Val, Ser or Thr;  Xaa13:

Gly or Glu

(xi)  SEQUENCE DESCRIPTION:  SEQ ID NO:  28

Phe Xaa Xaa Xaa Xaa Xaa Xaa Val Ala Xaa Xaa Xaa
1               5               10
Xaa Asp

SEQ ID NO:  29

(i)  SEQUENCE CHARACTERISTICS:

(A)  LENGTH:  14 amino acids

(B)  TYPE:  amino acid

(D)  TOPOLOGY:  linear

(ii)  MOLECULE TYPE:  peptide

(iii)  HYPOTHETICAL:  yes

(ix)  FERTURE

(D)  OTHER INFORMATION:

Xaa3:  Asp, Asn, Pro, Lys or Thr;  Xaa4,

Xaa5:  any amino acid;  Xaa6:  Gly, Glu or

Ser;  Xaa7:  Leu or Ile;  Xaa8:  Gly or

Thr;  Xaa9:  Asp, Lys or Thr;  Xaa10:

Val, Leu, Pro or Asn;  Xaa11:  Gly or Pro;

Xaa13:  Ser, Pro, Asp, Glu or Ile

(xi)  SEQUENCE DESCRIPTION:  SEQ ID NO:  29

```
Lys Leu Xaa Xaa Xaa Xaa Xaa Xaa Xaa Xaa Xaa Leu
 1               5                  10
Xaa Leu
```

SEQ ID NO:  30

  (i)  SEQUENCE CHARACTERISTICS:

    (A)  LENGTH:  14 amino acids

    (B)  TYPE:  amino acid

    (D)  TOPOLOGY:  linear

  (ii)  MOLECULE TYPE:  peptide

  (iii)  HYPOTHETICAL:  yes

  (ix)  FERTURE

    (D)  OTHER INFORMATION:

        Xaa2:  Lys or Phe;  Xaa3:  Asp or Gln;

        Xaa4:  Ile or Tyr;  Xaa5:  Gly, Asp or

        Tyr;  Xaa6:  Arg or Asn;  Xaa8:  Glu, Leu

        or Tyr;  Xaa9:  Asp, Lys, Glu or Ile;

        Xaa10:  Asp, Lys, Glu or Ile;  Xaa11:

        Asp, Ala, Val or Phe;  Xaa12:  Asp or Lys;

        Xaa13:  Val or Leu;  Xaa14:  Pro, Lys or

        Val

  (xi)  SEQUENCE DESCRIPTION:  SEQ ID NO:  30

```
Leu Xaa Xaa Xaa Xaa Xaa Glu Xaa Xaa Xaa Xaa Xaa
 1               5                  10
Xaa Xaa
```

SEQ ID NO:  31

  (i)  SEQUENCE CHARACTERISTICS:

    (A)  LENGTH:  14 amino acids

(B) TYPE: amino acid

(D) TOPOLOGY: linear

(ii) MOLECULE TYPE: peptide

(iii) HYPOTHETICAL: yes

(ix) FERTURE

(D) OTHER INFORMATION:

Xaa2: Asp, Ala or Ile; Xaa5: any amino acid; Xaa7: Ala or Asn; Xaa9: Val or Gly; Xaa10: Gly, Ser or Lys; Xaa13: Gly, Ala, Lys or Ile; Xaa14: Leu or Ala

(xi) SEQUENCE DESCRIPTION: SEQ ID NO: 31

```
Pro Xaa Lys Gly Xaa Thr Xaa Ala Xaa Xaa Lys Val
 1               5                   10
Xaa Xaa
```

SEQ ID NO: 32

(i) SEQUENCE CHARACTERISTICS:

(A) LENGTH: 11 amino acids

(B) TYPE: amino acid

(D) TOPOLOGY: linear

(ii) MOLECULE TYPE: peptide

(iii) HYPOTHETICAL: yes

(ix) FERTURE

(D) OTHER INFORMATION:

Xaa3, Xaa7: any amino acid; Xaa5: Asn, Val or Leu; Xaa6: Ser, Asn or Val

(xi) SEQUENCE DESCRIPTION: SEQ ID NO: 32

```
Tyr Val Xaa Leu Xaa Xaa Xaa Asn Leu Thr Asp
 1               5                   10
```

SEQ ID NO:  33

     (i)   SEQUENCE CHARACTERISTICS:

        (A)   LENGTH:  7 amino acids

        (B)   TYPE:  amino acid

        (D)   TOPOLOGY:  linear

    (ii)  MOLECULE TYPE:  peptide

   (iii)  HYPOTHETICAL:  yes

    (ix)  FERTURE

        (D)   OTHER INFORMATION:

           Xaa4, Xaa6:  any amino acid;  Xaa2:  Gly

           or Ser;  Xaa7:  Gly or Ala

    (xi)  SEQUENCE DESCRIPTION:  SEQ ID NO:  33

Cys Xaa Ala Xaa Gly Xaa Xaa
$\quad$1$\qquad\qquad$5

SEQ ID NO:  34

     (i)   SEQUENCE CHARACTERISTICS:

        (A)   LENGTH:  11 amino acids

        (B)   TYPE:  amino acid

        (D)   TOPOLOGY:  linear

    (ii)  MOLECULE TYPE:  peptide

   (iii)  HYPOTHETICAL:  yes

    (ix)  FERTURE

        (D)   OTHER INFORMATION:

           Xaa2, Xaa3, Xaa6, Xaa8, Xaa10:  any amino

           acid;  Xaa5:  Gly or Ser

    (xi)  SEQUENCE DESCRIPTION:  SEQ ID NO:  34

```
            His Xaa Xaa Ser Xaa Xaa Gly Xaa Gly Xaa Gly
             1               5                   10
```

SEQ ID NO: 35

    (i)  SEQUENCE CHARACTERISTICS:

    (A)  LENGTH:  11 amino acids

    (B)  TYPE:  amino acid

    (D)  TOPOLOGY:  linear

   (ii)  MOLECULE TYPE:  peptide

  (iii)  HYPOTHETICAL:  yes

   (ix)  FERTURE

    (D)  OTHER INFORMATION:

        Xaa3, Xaa4, Xaa5, Xaa7, Xaa9, Xaa10:  any

        amino acid;  Xaa11:  Gly, Ala or Ser

   (xi)  SEQUENCE DESCRIPTION:  SEQ ID NO:  35

```
      Arg Ser Xaa Xaa Xaa Al Xaa Ser Xaa Xaa Xaa
        1               5                   10
```

SEQ ID NO:  36

    (i)  SEQUENCE CHARACTERISTICS:

    (A)  LENGTH:  14 amino acids

    (B)  TYPE:  amino acid

    (D)  TOPOLOGY:  linear

   (ii)  MOLECULE TYPE:  peptide

  (iii)  HYPOTHETICAL:  yes

   (ix)  FERTURE

    (D)  OTHER INFORMATION:

        Xaa2, Xaa4, Xaa5, Xaa6, Xaa7, Xaa9, Xaa10,

        Xaa12, Xaa14:  any amino acid

(xi) SEQUENCE DESCRIPTION: SEQ ID NO: 36

Ala Xaa Ser Xaa Xaa Xaa Xaa Gly Xaa Xaa Gly Xaa
1               5                   10
Ala Xaa

SEQ ID NO: 37

(i) SEQUENCE CHARACTERISTICS:

(A) LENGTH: 13 amino acids

(B) TYPE: amino acid

(D) TOPOLOGY: linear

(ii) MOLECULE TYPE: peptide

(iii) HYPOTHETICAL: yes

(ix) FERTURE

(D) OTHER INFORMATION:

Xaa3, Xaa4, Xaa5: any amino acid; Xaa2:

Gly or Ser; Xaa11: Ala or Gly; Xaa13:

Gly or Ser

(xi) SEQUENCE DESCRIPTION: SEQ ID NO: 37

Gly Xaa Xaa Xaa Xaa Ser Gly Ala Ser Gly Xaa Gly
1               5                   10
Xaa

SEQ ID NO: 38

(i) SEQUENCE CHARACTERISTICS:

(A) LENGTH: 13 amino acids

(B) TYPE: amino acid

(D) TOPOLOGY: linear

(ii) MOLECULE TYPE: peptide

(iii) HYPOTHETICAL: yes

(ix) FERTURE

(D)   OTHER INFORMATION:

      Xaa2:  Gly or Ser;  Xaa5, Xaa8, Xaa10,

      Xaa12:  any amino acid

(xi)   SEQUENCE DESCRIPTION:  SEQ ID NO:  38

     Ser Xaa Ala Ser Xaa Gly Ser Xaa Gly Xaa Ser Xaa
      1              5                  10
     Gly

SEQ ID NO:  39

  (i)   SEQUENCE CHARACTERISTICS:

     (A)   LENGTH:  10 amino acids

     (B)   TYPE:  amino acid

     (D)   TOPOLOGY:  linear

 (ii)   MOLECULE TYPE:  peptide

(iii)   HYPOTHETICAL:  yes

 (ix)   FERTURE

     (D)   OTHER INFORMATION:

      Xaa2:  Gly or Ala;  Xaa6:  Ala or Ser;

      Xaa9:  Ala or Ser;  Xaa10:  Gly or Ser;

      Xaa4, Xaa7:  any amino acid

(xi)   SEQUENCE DESCRIPTION:  SEQ ID NO:  39

     Thr Xaa Ala Xaa Ala Xaa Xaa Ala Xaa Xaa
      1              5                  10

SEQ ID NO:  40

  (i)   SEQUENCE CHARACTERISTICS:

     (A)   LENGTH:  13 amino acids

     (B)   TYPE:  amino acid

     (D)   TOPOLOGY:  linear

 (ii)   MOLECULE TYPE:  peptide

(iii) HYPOTHETICAL:  yes

(ix) FERTURE

(D)  OTHER INFORMATION:

Xaa2, Xaa3, Xaa7, Xaa11, Xaa12:  any amino

acid;  Xaa5:  Gly or Ala;  Xaa8:  Ser or

Ala;  Xaa10:  Ser or Ala

(xi) SEQUENCE DESCRIPTION:  SEQ ID NO:  40

Asn Xaa Xaa Ser Xaa Ala Xaa Xaa Gly Xaa Xaa Xaa
1               5                    10
Ser

SEQ ID NO:  41

(i)  SEQUENCE CHARACTERISTICS:

(A)  LENGTH:  13 amino acids

(B)  TYPE:  amino acid

(D)  TOPOLOGY:  linear

(ii)  MOLECULE TYPE:  peptide

(iii) HYPOTHETICAL:  yes

(ix) FERTURE

(D)  OTHER INFORMATION:

Xaa4, Xaa7, Xaa8:  any amino acid;  Xaa3:

Leu or Lys;  Xaa13:  Gly or Lys

(xi) SEQUENCE DESCRIPTION:  SEQ ID NO:  41

Asp Pro Xaa Xaa Leu Xaa Xaa Val Lys Leu Leu Leu
1               5                    10
Xaa

SEQ ID NO:  42

(i)  SEQUENCE CHARACTERISTICS:

(A)  LENGTH:  13 amino acids

(B)   TYPE:  amino acid

(D)   TOPOLOGY:  linear

(ii)   MOLECULE TYPE:  peptide

(iii)   HYPOTHETICAL:  yes

(ix)   FERTURE

(D)   OTHER INFORMATION:

Xaa2, Xaa3, Xaa4, Xaa5, Xaa7, Xaa12:  any

amino acid;  Xaa10:  Asp or Leu;  Xaa11:

Gly or Asp;  Xaa13:  Gly or Lys

(xi)   SEQUENCE DESCRIPTION:  SEQ ID NO:   42

```
Glu Xaa Xaa Xaa Xaa Lys Xaa Leu Leu Xaa Xaa Xaa
  1               5                   10
Xaa
```

SEQ ID NO:   43

(i)   SEQUENCE CHARACTERISTICS:

(A)   LENGTH:   14 amino acids

(B)   TYPE:  amino acid

(D)   TOPOLOGY:  linear

(ii)   MOLECULE TYPE:  peptide

(iii)   HYPOTHETICAL:  yes

(ix)   FERTURE

(D)   OTHER INFORMATION:

Xaa6, Xaa9, Xaa10:  any amino acid;  Xaa2:

Gly or Leu;  Xaa5:  Gly or Val;  Xaa7:

Val or Asp;  Xaa11:  Gly or Leu

(xi)   SEQUENCE DESCRIPTION:  SEQ ID NO:   43

```
              Phe Xaa Asp Asp Xaa Xaa Xaa Val Xaa Xaa Xaa Val
                1                   5                   10
              Gly Asp
```

SEQ ID NO:  44

    (i)   SEQUENCE CHARACTERISTICS:

        (A)   LENGTH:  14 amino acids

        (B)   TYPE:  amino acid

        (D)   TOPOLOGY:  linear

    (ii)  MOLECULE TYPE:  peptide

  (iii)  HYPOTHETICAL:  yes

    (ix)  FERTURE

        (D)   OTHER INFORMATION:

           Xaa4, Xaa5:  any amino acid;  Xaa3:  Asp

           or Lys;  Xaa9:  Asp or Lys;  Xaa10:  Val

           or Leu

    (xi)  SEQUENCE DESCRIPTION:  SEQ ID NO:  44

```
              Lys Leu Xaa Xaa Xaa Gly Leu Gly Xaa Xaa Gly Leu
                1                   5                   10
              Asp Leu
```

SEQ ID NO:  45

    (i)   SEQUENCE CHARACTERISTICS:

        (A)   LENGTH:  14 amino acids

        (B)   TYPE:  amino acid

        (D)   TOPOLOGY:  linear

    (ii)  MOLECULE TYPE:  peptide

  (iii)  HYPOTHETICAL:  yes

    (ix)  FERTURE

        (D)   OTHER INFORMATION:

Xaa4, Xaa6, Xaa7, Xaa8: any amino acid;

Xaa5: Gly or Asp: Xaa10: Asp or Lys;

Xaa11: Asp or Val; Xaa12: Asp or Lys;

Xaa13: Val or Leu; Xaa14: Lys or Val

(xi) SEQUENCE DESCRIPTION: SEQ ID NO: 45

```
Leu Lys Asp Xaa Xaa Xaa Xaa Xaa Leu Xaa Xaa Xaa
 1               5                   10
Xaa Xaa
```

SEQ ID NO: 46

(i) SEQUENCE CHARACTERISTICS:

(A) LENGTH: 14 amino acids

(B) TYPE: amino acid

(D) TOPOLOGY: linear

(ii) MOLECULE TYPE: peptide

(iii) HYPOTHETICAL: yes

(ix) FERTURE

(D) OTHER INFORMATION:

Xaa5, Xaa6, Xaa7, Xaa8: any amino acid;

Xaa9: Val or Gly; Xaa10: Gly or Lys;

Xaa13: Gly or Lys

(xi) SEQUENCE DESCRIPTION: SEQ ID NO: 46

```
Pro Asp Lys Gly Xaa Xaa Xaa Xaa Xaa Xaa Lys Val
 1               5                   10
Xaa Leu
```

SEQ ID NO: 47

(i) SEQUENCE CHARACTERISTICS:

(A) LENGTH: 11 amino acids

(B) TYPE: amino acid

    (D)  TOPOLOGY:  linear

  (ii)  MOLECULE TYPE:  peptide

  (iii)  HYPOTHETICAL:  yes

  (ix)  FERTURE

      (D)  OTHER INFORMATION:

        Xaa3, Xaa7, Xaa8, Xaa10:  any amino acid;

        Xaa5:  Val or Leu

  (xi)  SEQUENCE DESCRIPTION:  SEQ ID NO:  47

    Tyr Val Xaa Leu Xaa Val Xaa Xaa Leu Xaa Asp


SEQ ID NO:  48

    (i)  SEQUENCE CHARACTERISTICS:

      (A)  LENGTH:  117 amino acids

      (B)  TYPE:  amino acid

      (D)  TOPOLOGY:  linear

  (ii)  MOLECULE TYPE:  protein

  (xi)  SEQUENCE DESCRIPTION:  SEQ ID NO:  48

    Thr Gly Cys Ala Phe Arg Ser Asp Glu Ser Val Arg
     1               5                   10

    Thr Lys Gln Asp Cys Gln Val Ala Glu Ser His His
                  15                  20

    Val Asn Xaa Xaa Thr Val Leu Arg Ser Val Gly Val
     25                  30                  35

    Cys Tyr Leu Val Gly Gly Gly Asp Cys Ser Leu Arg
                  40                  45

    Val Gly Ala Ala Val Xaa Leu Val Lys Ala Tyr Gln
          50                  55                  60

    Val Ile Tyr His Asn Val Cys Phe Ser Gly Xaa Leu
                  65                  70

```
Leu Cys Leu Leu Cys His Trp Phe Pro Asp Leu Ala
        75                  80

Leu Ala Ala Thr Gly Tyr Ser His Ser Thr Trp Lys
 85                  90                      95

Phe Glu Arg Pro Arg Leu Tyr Thr Met Arg Gly Glu
            100                 105

Ser Lys Leu Phe Xaa Pro Asp Thr Leu
    110             115
```

## Claims

1. A method for preparing a method for surmising the functional site or region in a physiologically active polypeptide whose amino acid sequence is known (test polypeptide), which comprises

   (1) selecting, as the reference polypeptides, a plurality of known physiologically active peptides whose action site amino acid is known; supposing, as the functional regions in the known amino acid sequence of these reference polypeptides (reference amino acid sequence), the suposed functional regions which consist of a plurality of amino acids in both downstream and upstream of the active site amino acid, and the supposed non-functional region excluding a functional region;

   (2) supposing many amino acid sequence patterns (referred to as supposed amino acid sequence patterns) conforming to the following formula (I) consisting of two or more amino acids selected from 20 amino acids usually existing in natural physiologically active polypeptides:

   $$X_1 (Z)_n X_2 \qquad (I)$$

   (wherein $X_1$ and $X_2$ are the same or different, and are each any specific amino acid, $Z_s$ are the same or different unspecific amino acids, n is 0 or an integer of up to about 30, and $X_1$ is here referred to as a reference amino acid);

   (3) scanning, by each of the many supposed amino acid sequence patterns of the above (2), the reference amino acid sequences selected in the above (1) with the supposed functional region and/or the supposed non-functional region; selecting supposed amino acid sequence patterns having a high frequency in accordance with the supposed functional region and/or supposed amino acid sequence patterns having a high frequency in accordance with the supposed non-functional region; and with respect to each supposed region, superposing, the supposed amino acid sequence patterns which have same reference amino acid $X_1$ wherein the reference amino acid $X_1$ is rearranged to determine an amino acid sequence pattern related to the supposed functional region (supposed functional region-related amino acid sequence pattern) or an amino acid sequence pattern related to the supposed non-functional region (supposed non-functional region-related amino acid sequence pattern) (both are all-inclusively referred to as a related amino acid sequence pattern); and

   (4) determining symmetrical related amino acid sequence patterns consisting of the supposed functional and non-functional region-related amino acid sequence patterns determined in the above (3), which have a small number of amino acids extending symmetrically in the direction upstream and downstream of the reference amino acid $X_1$ as a center; comparing the symmetrical related amino acid sequence pattern: with the reference amino acid sequences selected in the above (1) by superposing; determining the number of amino acids between both sequences in the range of the predetermined number of amino acids containing the amino acid as the center (identical amino acids), and comparing the results with the supposed functional region and/or supposed non-functional region in the reference amino acid sequences, to prepare a criterion for the determination of the functional sites or regions.

2. A method for identifying the functional site or region of a polypeptide whose amino acid sequence is known but whose functional site or region is not known (test polypeptide), which comprises comparing the amino acid sequence of the test polypeptide with the symmetrical related amino acid patterns determined in the (4) of claim 1, and applying to the results the criterion determined in the (4) of claim 1.

3. A method for preparing a method for surmising a functional site or region in a physiologically active polypeptide whose amino acid sequence is known (test polypeptide), which comprises

(1) selecting, as the reference polypeptides, a plurality of known physiologically active peptides whose action site amino acid is known; supposing, as the functional regions in the known amino acid sequence of these reference polypeptides (reference amino acid sequence), the supposed functional regions which consist of a plurality of amino acids in both downstream and upstream of the active site amino acid, and the supposed non-functional region excluding a functional region;

(2) supposing many amino acid sequence patterns (referred to as secondary supposed amino acid sequence patterns) conforming to the following formula (II):

$$X_1' \, (Z)_m \, X_2' \qquad (II)$$

(wherein $X_1'$ and $X_2'$ are the same or different, and are each any specific amino acid, $Z_s$ are the same or different unspecific amino acids, and m is 0 or an integer of up to about 30, wherein, $X_2'$ is the same with $X_1'$, or any of the amino acid supposed when the genetic codon encoding $X_1'$ were read in the reverse direction of $3' \rightarrow 5'$, the amino acid supposed when the antisense corresponding to the codon was read in the direction of $5' \rightarrow 3'$ and the amino acid supposed when the antisense corresponding to the codon was read in the reverse direction of $3' \rightarrow 5'$); and

(3) carrying out multivariate analysis for inducing a discriminant function from the reference amino acid sequences to distinguish the supposed functional region from supposed non-functional region of the above (1) by the secondary supposed amino acid sequence patterns determined in the above (2).

4. A method for surmising the functional site or region in a physiologically active polypeptide whose amino acid sequence is known (test polypeptide), which comprises applying the discriminant function determined in the step (3) of claim 3 to the amino acid sequence of the test polypeptide.

5. A method for surmising a site or a region in a polynucleotide, encoding the functional site or region in a physiologically active polypeptide, which comprises applying the operations described in claim 1 or 2 to an amino acid sequence translated from the nucleotide sequence of the polynucleotide.

6. A method for surmising a site or a region in a polynucleotide, encoding the action site or region in a physiologically active polypeptide, which comprises applying the operations described in claim 3 or 4 to an amino acid sequence translated from the nucleotide sequence of the polynucleotide.

7. A synthetic polypeptide composed using at least two successive amino acids in the amino acid sequence of the functional site or region surmised by claim 2.

8. A synthetic polypeptide using at least two successive amino acids in the amino acid sequence of the functional site or region surmised by claim 4.

9. A synthetic polynucleotide using at least six successive nucleotides in the nucleotide sequence of the site or region of a polynucleotide surmised by claim 5.

10. A synthetic polynucleotide composed using at least six successive nucleotides in the nucleotide sequence of the site or region of a polypeptide surmised by claim 6.

11. A method of preparing a polypeptide or polynucleotide comprising performing a method according to any of claims 1-6 in order to surmise a sequence of a functional site or region of a physiologically active polypeptide or polynucleotide; and synthesising a polypeptide or a polynucleotide including at least part of said sequence, or a polynucleotide encoding at least part of said polypeptide sequence.

EP 0 494 502 A1

# Fig. 1

PEAK  AMINO  ACID  90'Ala'  137'Glu'

# Fig. 2

PEAK AMINO ACID 65'Tyr' 133'Pro' 220'Cys'

EP 0 494 502 A1

# Fig. 3

PEAK AMINO ACID 19'Asn' 179'His' 256'Asn' 348'Phe' 404'Trp'

# Fig. 4

sum

PEAK AMINO ACID 10'Asp' 57'Ala' 120'His' 248'Pro'

# Fig. 5

PEAK AMINO ACID 62'Gly' 128'Gly'

EP 0 494 502 A1

Fig. 6

sum

PEAK AMINO ACID 44'Pro' 86'Ile' 175'Thr' 230'Leu'

RESIDUE NUMBER

# Fig. 7

PEAK AMINO ACID  47'Cys'  125'Gln'  172'Leu'  222'Ala'  285'Glu'

# F I g. 8

PEAK AMINO ACID 11'Gly' 87'Ser' 175'Ala' 278'Lys'

EP 0 494 502 A1

# Fig. 9

PEAK AMINO ACID 75'His' 263'Arg' 425'Cys' 488'Asp'

# F l g. 10

PEAK AMINO ACID 42'Ser' 127'Val'

EP 0 494 502 A1

# Fig. 11

PEAK AMINO ACID 23'Gly' 65'Cys' 159'Ala'

F i g. 12

PEAK AMINO ACID 110'Ala' 147'Ile' 211'Thr' 318'Phe'

EP 0 494 502 A1

EP 0 494 502 A1

# F I g. 13

PEAK AMINO ACID 18'Gly' 61'Asp' 126'Ile'

EP 0 494 502 A1

# F l g. 14

sum

PEAK AMINO ACID 19'Ala' 194'Tyr' 383'Ala' 465'Gly

RESIDUE NUMBER

(FURTHER CONTINUE RIGHT )

# F I g. 15

sum

PEAK AMINO ACID 19'Val' 66'Val'

**EP 0 494 502 A1**

European Patent Office

**EUROPEAN SEARCH REPORT**

Application Number

EP 91 31 1129

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | PROTEIN ENGINEERING. vol. 2, no. 2, 1988, EYNSHAM, OXFORD, ENGLAND GB pages 127 - 138; M.J.J.M. ZVELEBIL ET AL.: 'Analysis and prediction of the location of catalytic residues in enzymes' * page 132, left column, paragraph 2 - page 133, left column, paragraph 1 * | 1 | G01N33/68 C07K3/00 |
| D,A | MEDICAL HYPOTHESES vol. 7, no. 8, August 1981, EDINBURGH, UK pages 981 - 983; J. BIRO: 'Comparative analysis of specificity in protein-protein interactions; Part II' * page 981, paragraph 2 - page 982, paragraph 3 * * page 992, paragraph 2 - page 993, paragraph 4 * | 1 | |
| | | | TECHNICAL FIELDS SEARCHED (Int. Cl.5 ) |
| | | | G01N |

The present search report has been drawn up for all claims

| Place of search | Date of completion of the search | Examiner |
|---|---|---|
| THE HAGUE | 04 FEBRUARY 1992 | THIELE U.H.-C.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)